# EUROPÄISCHE PATENTSCHRIFT

(11) **EP 3 171 919 B1**
(45) Veröffentlichungstag und Bekanntmachung des Hinweises auf die Patenterteilung: **12.09.2018**
(21) Anmeldenummer: 16739186.1
(22) Anmeldetag: 18.07.2016
(51) Int. Cl.: A61M 5/32, A61M 25/06

(54) **SICHERHEITSKANÜLE**
SECURITY CANNULA
CANULE DE SÉCURITÉ

(30) Priorität: 16.07.2015 DE 102015009190
(43) Veröffentlichungstag der Anmeldung: 31.05.2017
(60) Teilanmeldung: 18183192.6
(73) Patentinhaber: B. Braun Avitum AG, 34212 Melsungen (DE)
(72) Erfinder: HAINDL, Hans, 30974 Wennigsen (DE); WOEHR, Kevin, 34587 Felsberg (DE)
(74) Vertreter: Winter, Brandl, Fürniss, Hübner, Röss, Kaiser, Polte - Partnerschaft mbB
(86) Internationale Anmeldenummer: PCT/EP2016/067044
(87) Internationale Veröffentlichungsnummer: WO 2017/009483

(56) Entgegenhaltungen:
- DE-A1-102012 206 557
- US-A1- 2002 004 650
- US-A1- 2015 151 086

## Beschreibung

Die vorliegende Anmeldung betrifft eine Sicherheitskanüle für die extrakorporale Blutbehandlung wie zum Beispiel für die Dialyse, insbesondere eine Dialysekanüle oder eine Fistula-Kanüle.

Die Unfallverhütungsvorschrift TRBA 250 fordert, dass an allen medizinischen Arbeitsplätzen, bei denen eine Verletzungsgefahr für Anwender von der Handhabung von Kanülen ausgeht, soweit verfügbar, Sicherheitskanülen eingesetzt werden, die nach Gebrauch aktiv oder passiv so geschützt sind, dass sie den Anwender oder Dritte nicht mehr verletzen können. Diese Forderung gilt unter anderem auch für Dialyse-Stationen und Dialyse-Zentren. Bei der Dialyse wird ein künstlich unter der Haut angelegtes Gefäß, ein sogenannter Shunt, zumeist mit zwei Kanülen punktiert. Diese Kanülen sind großlumig und enthalten daher relativ große Blutmengen, so dass bei der Verletzung des Anwenders mit diesen Kanülen die Infektionsgefahr als besonders hoch einzuschätzen ist. Dazu kommt, dass die Häufigkeit durch Blut übertragbarer Infektionen, wie etwa Hepatitis B und C sowie HIV, unter Dialyse-Patienten deutlich höher ist als im Durchschnitt der Bevölkerung.

Um die Infektionsgefahr hier zu vermindern, wurden bereits sogenannte Sicherheitskanülen für die Dialyse vorgeschlagen. Das Prinzip dieser Sicherheitskanülen beruht darauf, dass nach Gebrauch der Kanüle, jedoch vor dem Entfernen des mit der Haut des Patienten verklebten Kanülenansatzes die Kanüle durch Ziehen am Schlauchansatz der Kanüle aus dem Blutgefäß entfernt wird und in den Kanülenansatz hineingezogen wird. Dort rastet die Kanüle dann ein, so dass die Kanülenspitze nicht wieder aus dem Kanülenansatz hervortreten kann.

Bedauerlicherweise hat es mit diesen Sicherheitskanülen schwerwiegende Zwischenfälle gegeben, die dadurch entstanden sind, dass der Schlauch an der Kanüle nicht wie vom Hersteller gefordert - separat mit Pflaster auf der Haut des Patienten befestigt worden ist. Dadurch konnten z.B. durch Bewegung des Patienten Zugkräfte auf den Schlauch wirken, was dazu führen konnte, dass die Kanüle während der Dialyse in ihre Schutzvorrichtung zurückgezogen wurde. Dadurch wurde bei der venösen Kanüle das Blut aus der Dialysemaschine nicht in den Patienten zurückgefördert, sondern in die Umgebung. Da der Ausfluss des Blutes nicht behindert wird und es damit zu keiner Drucksteigerung im venösen Schlauchschenkel, auch als venöse Seite bezeichnet, kommt, pumpt die Dialysemaschine in einem solchen Fall weiter, ohne Alarm zu geben. Dies kann zum Verbluten des Patienten führen.

Die aufgetretenen Zwischenfälle mit den Sicherheitskanülen beleuchten ein Problem der Dialyse, das von Beginn an bestanden hat und bis heute nicht gelöst ist. Dies ist die Sicherheitsüberwachung des venösen Schenkels der Dialyse. Die Dialysemaschinen sind mit sensiblen Sensoren versehen, die Störungen im Ablauf der Dialyse wahrnehmen und die Maschine gegebenenfalls abschalten und/oder Alarm geben. Wenn etwa die arterielle Kanüle herausrutscht, so wird zunächst, solange die Kanülenspitze noch im Gewebe liegt, die Kanüle kein Blut mehr ansaugen können, und es kommt zum Unterdruck im arteriellen Schlauchschenkel, auch als arterielle Seite bezeichnet. Dies registriert die Dialysemaschine, sie schaltet sich ab und gibt Alarm. Rutscht die arterielle Kanüle komplett aus dem Gewebe heraus, so zieht sie Luft statt Blut. Dies wird in der Maschine sofort z.B. durch einen Ultraschall-Luftdetektor bemerkt, die Maschine schaltet sich aus und gibt Alarm. Insofern ist der arterielle Schenkel automatisch überwacht.

Anders ist dies beim venösen Schlauchschenkel. Wenn die venöse Kanüle verrutscht und mit der Spitze im Gewebe, also zwischen Shunt und Haut liegt, wird sie durch den gepumpten Blutstrom sofort komplett aus dem Gewebe ausgeworfen, und der Blutstrom entleert sich in die Umgebung. Dabei treten in der Regel nur so kleine und kurzfristige Druckänderungen auf, dass der Drucksensor des Dialyse-Gerätes diese häufig nicht wahrnehmen kann. Deshalb hat es durch das Herausrutschen der venösen Kanüle auch schon zahlreiche Todesfälle durch Verbluten der Dialyse-Patienten gegeben. Bei den verwendeten Förderraten kann der Patient nach drei bis fünf Minuten tot sein.

Im Stand der Technik sind verschiedene Verfahren und Vorrichtungen beschrieben, mittels derer der korrekte Sitz einer Kanüle überwacht werden kann, vgl. z.B. DE 10 2009 004 018 A1, US 2006/0130591 A1, DE 199 53 068 A1 und WO 99/24145. Diese Verfahren und Vorrichtungen sind jedoch ausgesprochen komplex und verlangen insbesondere nach einer speziellen Sensorik. Dementsprechend sind diese bekannten Verfahren und Vorrichtungen teuer und wenig praktikabel.

Ferner ist aus der DE 10 2012 206 557 A1 eine Sicherheitskanüle bekannt, welche eine Sicherheitseinrichtung aufweist, die dazu geeignet ist, bei Ablösen des Kanülengehäuses von der Haut des Patienten automatisch die Punktionskanüle in eine geschützte Position in das Gehäuse zurückzuziehen. Eine distale Öffnung des Kanülengehäuses kann dabei mit Hilfe eines Abdeckelements teilweise verschlossen werden, um eine Hemmung des Blutflusses zu bewirken, der einen von einem Dialysegerät registrierbaren Druckanstieg verursacht. Allerdings wird bei dieser Sicherheitskanüle die Kanüle durch die Sicherheitseinrichtung aktiv bewegt, was insofern nachteilig sein kann, als sie dann unter Umständen aus dem Blutgefäß gezogen wird, selbst wenn sie noch ordnungsgemäß darin lag. Darüber hinaus ist der Verschlussmechanismus mit Hilfe des zusätzlichen Abdeckelements relativ kompliziert, da er einerseits eines Mechanismus zum Schutz der Kanüle und andererseits eines Mechanismus zum Generieren des Blutstaus bedarf.

Weiterhin offenbart die US 2015/151086 A1 eine Sicherheitskanüle, welche an einem Katheter vorgesehen ist. Wird die Kanüle bzw. die Nadel dabei von einem Anwender in ein Kathetergehäuse zurückgezogen, bedeckt ein sich darin befindlicher Nadelschutz die Nadel-/ bzw. Kanülenspitze.

Es ist daher eine Aufgabe der vorliegenden Erfindung, eine Sicherheitskanüle bereitzustellen, die den oben genannten Sicherheitsanforderungen auf einfache Weise, aber dennoch zuverlässig Rechnung trägt. Es ist eine weitere Aufgabe der vorliegenden Erfindung, eine Sicherheitskanüle bereitzustellen, die kostengünstig herstellbar ist und in ihrer Anwendung einfach handhabbar ist.

Diese und weitere Aufgaben werden mit einer Sicherheitskanüle gemäß Anspruch 1 gelöst. Weitere bevorzugte Merkmale sind in den abhängigen Ansprüchen beschrieben.

Entsprechend stellt die vorliegende Erfindung eine Sicherheitskanüle, eine Haltevorrichtung und eine Sicherheitseinrichtung bereit. Die Punktionskanüle weist ein Kanülenlumen und eine, bevorzugt angeschliffene, Spitze auf. Die Haltevorrichtung ist dazu geeignet, während des Gebrauchs in einer definierten Position an der Haut eines Patienten anzuliegen und die Punktionskanüle zu halten. Die Sicherheitseinrichtung weist ein Schutzelement auf, welches einen ersten, geöffneten Zustand und einen zweiten, geschlossenen Zustand einnehmen kann. Die Sicherheitseinrichtung ist bevorzugt dazu geeignet, bei Ablösen der Haltevorrichtung von der Haut und/oder beim Bewegen der Haltevorrichtung gegenüber der definierten Position automatisch das Schutzelement aus einer ersten Position, in der die Spitze der Kanüle freiliegt, in eine zweite Position, in der die Spitze der Kanüle abgedeckt ist, zu bewegen, wodurch gleichzeitig das Schutzelement automatisch oder selbstaktivierend vom ersten, geöffneten Zustand in den zweiten, geschlossenen Zustand übergeht. Bevorzugt wird die Sicherheitseinrichtung bei Ablösen der Haltevorrichtung von der Haut und/oder beim Bewegen der Haltevorrichtung gegenüber der definierten Position automatisch in der ersten Position aktiviert, d.h. in einen Zustand versetzt, in dem sich das Schutzelement aus der ersten in die zweite Position bewegen kann, sofern eine solche Bewegung nicht dadurch blockiert wird, dass sich die Kanülenspitze noch immer in dem Gefäß befindet. Sobald dann die Kanüle aus dem Gefäß herausrutscht und die Spitze der Kanüle freiliegt, bewegt sich das Schutzelement bevorzugt automatisch in die zweite Position, in der die Spitze der Kanüle abgedeckt ist. Dementsprechend wird eine Verletzungsgefahr durch die Kanülenspitze wirksam minimiert, ohne dass die erfindungsgemäße Sicherheitseinrichtung versehentlich zu einem Herausziehen der Kanüle aus dem Gefäß führen würde.

Mit anderen Worten ist die erfindungsgemäße Sicherheitskanüle bevorzugt derart ausgestaltet, dass sich die Kanüle nicht mit offenliegender Spitze vom Patienten lösen kann, da ein Ablösen der Haltevorrichtung von der Haut und/oder ein Bewegen der Haltevorrichtung gegenüber der definierten Position die Punktionskanüle zusammen mit der angeschliffenen Spitze sichert. Dabei wird die Kanüle von der Sicherheitseinrichtung bevorzugt nicht aktiv bewegt. Vielmehr erfolgt das Sichern bevorzugt dadurch, dass das Schutzelement aus der ersten Position in die zweite Position bewegt wird. Dies ist insbesondere dann von Vorteil, wenn die Sicherheitseinrichtung nicht durch ein Ablösen der Haltevorrichtung von der Haut, sondern durch ein Bewegen der Haltevorrichtung gegenüber der definierten Position ausgelöst wird, da hier ein aktives Zurückziehen der Kanüle möglicherweise gar nicht nötig wäre, solange die Kanülenspitze noch im Gefäß liegt.

Mit noch anderen Worten ausgedrückt betrifft die vorliegende Erfindung gemäß einem ggf. unabhängig zu beanspruchenden Aspekt eine Punktionskanüle, eine Haltevorrichtung und eine Sicherheitseinrichtung, die gemeinsam eine Sicherheitskanüle gemäß der Erfindung bilden. Die Sicherheitseinrichtung ist dafür angepasst, in einem ersten, geöffneten Zustand eine Spitze der Punktionskanüle freiliegend zu halten (erste Rastposition) und in einen zweiten, geschlossenen Zustand relativ zur Punktionskanüle bewegt zu werden (z.B. durch eine Feder), in welchem die Spitze der Punktionskanüle zur Umgebung hin abgeschirmt/abgedeckt und vorzugsweise zur Umgebung hin zumindest teilweise verschlossen ist (zweite Rastposition).

Darüber hinaus sind die Sicherheitseinrichtung und die Halteeinrichtung derart (konstruktiv) aufeinander abgestimmt, dass ein Ablösen der Haltevorrichtung von der Patientenhaut und/oder ein Bewegen/Verschieben der Haltevorrichtung gegenüber einer definierten Position auf der Patientenhaut die Sicherheitseinrichtung in einen ausgelösten/scharf geschalteten Zwischenzustand versetzt, in welchem deren Bewegungsmechanismus zum Überführen der Sicherheitseinrichtung vom ersten in den zweiten zustand in Funktion gesetzt ist (dieser aber erst zur Wirkung kommt, sobald die Punktionskanüle aus dem Patienten-Blutgefäß quasi als Widerlager aktiv gezogen wird.

In einer bevorzugten Ausführungsform weist die Sicherheitseinrichtung eine Schutzelementbewegungsvorrichtung (Bewegungs-Mechanismus) auf, mithilfe derer das Schutzelement aus der ersten in die zweite Position bewegt werden kann. Bevorzugt ist diese Schutzelementbewegungsvorrichtung proximal zu dem Schutzelement angeordnet. Bevorzugt hat die Schutzelementbewegungsvorrichtung eine erste Feder und eine Haltewand oder ein Haltering. Bevorzugt ist das Schutzelement nicht mit dem Gehäuse der Haltevorrichtung verbunden, bevorzugt nicht materialschlüssig verbunden.

Die vorliegende Erfindung ist grundsätzlich für alle jene Kanülen von Bedeutung, die ein Gehäuse oder eine Haltevorrichtung aufweisen, das/die vorübergehend in Kontakt mit der Haut eines Patienten gebracht wird. Insbesondere ist jedoch die Sicherheitskanüle der vorliegenden Erfindung für die extrakorporale Blutbehandlung geeignet. Ein besonders bevorzugtes Anwendungsgebiet stellt aus den oben genannten Gründen die Dialyse dar.

Im Falle der Dialyse oder einer anderen extrakorporalen Blutbehandlung ist die Sicherheitskanüle der vorliegenden Erfindung jedoch besonders vorteilhaft, da das Schutzelement der Sicherheitseinrichtung im zweiten, geschlossenen Zustand den Blutstrom durch die Kanüle behindert. Dadurch erhöht sich der Druck im venösen Schlauchschenkel, was durch beispielsweise eine Dialysemaschine registriert werden kann, sodass sich diese automatisch abschaltet. Erfindungsgemäß ist hierfür (anders als bei der DE 10 2012 206 557 A1) kein zusätzliches Abdeckelement notwendig, welches eine Gehäuseöffnung verschließen würde, sondern das Schutzelement selbst kann auf- bzw. zugeklappt werden. Mit anderen Worten weist das Schutzelement eine Wandung auf, welche eine gehäuseartige Struktur bildet, wobei der geschlossene Zustand ausschließlich durch Elemente der Wandung erzielt wird. Dies kann beispielsweise dadurch erreicht werden, dass das Schutzelement zwei Halbschalen aufweist, die auseinander- bzw. zusammengeklappt werden können. Alternativ kann das Schutzelement zwei seitliche Wandabschnitte aufweisen, die gegen eine Federkraft derart nach außen bewegt werden können, dass der geöffnete Zustand hergestellt wird. Bevorzugt sind diese seitlichen Wandabschnitte elastisch ausgebildet und weisen an ihrem distalen Ende distale Wandabschnitte auf. Alternativ können auch vier seitliche Wandungsabschnitte elastisch ausgebildet sein, die alle aufeinander zu federn, um den geschlossenen Zustand zu bilden.

Bevorzugt weist das Schutzelement zwei federnde Arme auf, die das Schutzelement im zweiten Zustand verschließen. Diese zwei federnden Arme bilden bevorzugt, wie oben beschrieben, zwei Seitenwände eines durch das Schutzelement gebildeten Gehäuses. Dabei sind die Arme bevorzugt derart ausgebildet, dass sie im ersten Zustand reibungsarm über die Außenseite der Kanüle gleiten können.

In anderen Worten ausgedrückt hat die erfindungsgemäße Sicherheitseinrichtung bevorzugt ein weiter vorzugsweise Gehäuse-/Hülsen-/Klammer-förmiges Schutzelement, dessen distaler Endabschnitt zu zumindest einem (vorzugsweise zwei) elastisch vorgespannten Türflügel(n) ausgebildet ist, die von der Punktionskanüle in radialer Richtung aufgestoßen wird (werden) und bei eine Relativbewegung in Richtung distal über die Spitze der Kanüle hinaus selbsttätig zuklappt (zuklappen).

Bevorzugt weist mindestens einer der Arme/Türflügel an seinem distalen Ende eine Arretiereinrichtung auf, die verhindert, dass das Schutzelement aus der zweiten Position in die erste Position bewegt wird. Dabei kann die Arretiereinrichtung bevorzugt einen abgewinkelten Abschnitt des Arms und/oder einen Widerhaken aufweisen. Der abgewinkelte Abschnitt des Arms bildet dabei bevorzugt einen Teil einer distalen Wand des durch das Schutzelement gebildeten Gehäuses. Dabei ist es bevorzugt, dass der abgewinkelte Abschnitt des Arms bzw. die distale Wand des Schutzelements sowohl in radiale als auch in proximale Richtung weist. Mit anderen Worten beträgt der Winkel zwischen dem abgewinkelten Abschnitt des Arms und dem übrigen Arm bevorzugt weniger als 90°, stärker bevorzugt weniger als 80°, noch stärker bevorzugt weniger als 70° und besonders bevorzugt weniger als 60°. Ein solcher spitzer Winkel hat mehrere Vorteile. Einerseits erleichtert ein solcher spitzer Winkel das Gleiten der Arme über die Außenseite der Kanüle in distaler Richtung. Zum anderen kann ein solcher spitzer Winkel als Arretiereinrichtung dienen, da die Kanülenspitze bei dem Versuch, das Schutzelement in proximaler Richtung von der Kanüle zurückzuziehen, mit dem abgewinkelten Abschnitt des Arms in Eingriff tritt bzw. mit diesem verhakt.

Anstelle solcher abgewinkelter Arme kann das distale Ende des Schutzelements auch auf andere Weise konkav ausgebildet sein. Die obengenannten Vorteile werden in der Regel auch dann erreicht, wenn sich der distale Wandabschnitt proximal wölbt oder anders in proximale Richtung erstreckt, d.h., wenn das distale Ende des Schutzelements in das Innere des Schutzelements vorsteht.

Es ist ferner bevorzugt, dass die beiden Arme/Türflügel derart ausgebildet sind, dass sie (an ihren freien Endkanten) miteinander in Eingriff treten können, bevorzugt miteinander verrasten und/oder sich verhaken, wenn das Schutzelement in der zweiten, geschlossenen Position ist. Hierzu weisen die beiden Arme bevorzugt Hakenelemente bzw. gekrümmte Bereiche auf, die in der zweiten Position miteinander in Eingriff treten bzw. sich ineinander verhaken. Dadurch kann verhindert werden, dass die beiden Arme durch den Druck des Blutes auseinandergedrückt werden können.

Ferner besteht die Möglichkeit, einen der beiden Arme/Türflügel schmaler/kürzer zu gestalten als der andere, gegenüberliegende Arm/Türflügel und darüber hinaus für beide Arme/Türflügel einen Schwenk-Endanschlag am Gehäuse-förmigen Schutzelement auszubilden. Im Fall einer solche konstruktiven Ausgestaltung wird bei einem Verschieben der Gehäuse-förmigen Schutzelements relativ zur Punktionskanüle über deren Spitze hinaus zuerst der schmalere/kürzere Arm in die Schließposition verschwenken und anschließend (zeitlich versetzt) der breitere/längere Arm, sodass sich in Schließposition der beiden Arme diese dichtend übereinander legen, während sie sich am Endanschlag abstützen.

Bevorzugt besteht das Schutzelement zumindest abschnittsweise aus einem elastischen Kunststoff oder ist mit einem elastischen Kunststoff beschichtet, wobei der elastische Kunststoff bevorzugt eines oder eine beliebige Kombination der folgenden Materialien aufweist: Silikon, Polyurethan, PTFE. Ein solcher elastischer Kunststoff verbessert die Abdichtung zwischen den einzelnen Abschnitten des Schutzelements, d.h. zwischen den beiden Halbschalen oder den seitlichen Wänden des Schutzelements. Weist das Schutzelement zwei federnde Arme auf, ist es besonders bevorzugt, dass mindestens die Seitenränder dieser federnden Arme den genannten elastischen Kunststoff aufweisen. Alternativ weist das Schutzelement Metall, bevorzugt Edelstahl, bevorzugt in Form eines Bleches, auf, bevorzugt besteht es daraus.

Bevorzugt ist die Innenseite des Schutzelements zumindest abschnittsweise mit einem gerinnungsfördernden Material beschichtet, wobei das gerinnungsfördernde Material bevorzugt eines oder eine beliebige Kombination der folgenden Materialien aufweist: Proteine wie Kollagen, Fibrin, Thrombin; Polypeptide wie Gelatine; Polysacharide wie Cellulose, Zucker; Glucosamine wie Chitosan; Alginate; Adsorbierende Substanzen wie Zeolith, Aluminophosphat; Denaturierende Substanzen wie Aldehyde, Alaun, Aluminiumsalze. Eine solche Beschichtung hat den Vorteil, dass der Blutfluss nach dem Auslösen der Sicherheitseinrichtung zusätzlich gehemmt oder unterbunden wird. Während des Einsatzes der Sicherheitskanüle strömt Blut lediglich durch das Kanülenlumen. Wird jedoch die Kanülenspitze versehentlich aus der Vene des Patienten entfernt, sodass die Sicherheitseinrichtung ausgelöst wird und das Schutzelement in die zweite Position, in der die Spitze der Kanüle abgedeckt ist, bewegt wird, so pumpt die Dialysemaschine Blut in das Innere des Schutzelements. Gerinnt dieses aufgrund des gerinnungsfördernden Materials auf der Innenseite des Schutzelements, so wird der Strömungswiderstand deutlich erhöht, sodass ein vom Dialysegerät registrierbarer Druckanstieg in kürzerer Zeit erfolgen kann.

Ein ähnlicher Effekt kann dadurch erzielt werden, dass das Schutzelement auf der Innenseite eines oder einer beliebigen Kombination der folgenden Materialien aufweist: elastischer Schaumstoff wie zum Beispiel Schaumstoff aus Polyurethan oder Silikon; Quellstoffe; schwammartige und/oder wasserabsorbierende Substanzen. So kann beispielsweise ein im Inneren des Schutzelements vorgesehener elastischer Schaumstoff beim Verschließen des Schutzelements den Innenraum im Wesentlichen vollständig ausfüllen, sodass jeglicher Blutfluss wirksam unterbunden werden kann. Sind Quellstoffe auf der Innenseite des Schutzelements vorgesehen, so werden diese aufquellen, sobald sie mit Blut in Kontakt geraten, und dann ebenfalls den Innenraum des Schutzelements im Wesentlichen vollständig ausfüllen, um so eine wirkungsvolle Barriere für den Blutfluss darzustellen.

Bevorzugt weist die Kanüle einen, zwei oder mehrere Vorsprünge auf, der/die verhindern, dass sich das Schutzelement von der Kanüle löst. Wenn die erfindungsgemäße Sicherheitseinrichtung auslöst, wird im Falle der Dialyse Blut von dem Dialysegerät in das Innere des Schutzelements gepumpt. Würde sich aufgrund des dadurch entstehenden Drucks das Schutzelement von der Kanülenspitze lösen, so wäre die Sicherheitseinrichtung im Wesentlichen wirkungslos. Daher ist es bevorzugt, ein solches Ablösen mithilfe eines oder mehrerer Vorsprünge zu verhindern. Selbstverständlich kann die Kanüle auch andere Mittel zum Blockieren des Schutzelements gegen ein Verschieben in distaler Richtung aufweisen. So können am distalen Ende der Kanüle anstelle von Vorsprüngen auch ein oder mehrere aufgeweitete Bereiche oder eine Nut vorgesehen sein, in die das Schutzelement einrastet. Alternativ oder zusätzlich kann das Schutzelement auch durch ein Verbindungselement wie einen Faden mit dem Kanülenansatz (oder einem proximalen Abschnitt der Kanüle) verbunden sein. Der Faden hat dann bevorzugt eine Länge, die ein vollständiges Abstreifen des Gehäuse-/Klammer-förmigen Schutzelements über die Kanülenspitze verhindert.

Bevorzugt sind die zwei oder die mehreren Vorsprünge in axialer Richtung gesehen an der gleichen Position angeordnet. Bevorzugt sind mindestens zwei, bevorzugt genau zwei Vorsprünge in axialer Richtung gesehen an der gleichen Position angeordnet. Alternativ sind die zwei oder die mehreren Vorsprünge bevorzugt in axialer Richtung gesehen an verschiedenen Positionen angeordnet. Bevorzugt wird der eine, die zwei oder die mehreren Vorsprünge durch eine, bevorzugt mechanische, Quetschung erzeugt.

Bevorzugt ist an der Haltevorrichtung eine lösbare Einrichtung vorgesehen, um ein Auslösen des Sicherheitsmechanismus vor Gebrauch der Sicherheitskanüle zu verhindern. Dabei kann es sich bevorzugt um einen Klebestreifen und/oder einen lösbaren Clip und/oder Haltegriff handeln.

In einer bevorzugten Ausführungsform weist die Haltevorrichtung auf der der Haut des Patienten zugewandten Seite, also auf der dem Gehäuse der Sicherheitsvorrichtung abgewandt liegenden Seite, einen Klebestreifen auf. Der Klebestreifen ist vor der Verwendung, bevorzugt bis zur gewünschten Positionierung der Kanüle in der Vene mit einer Abdeckung, bevorzugt aus Papier, zumindest teilweise, bevorzugt vollständig abgedeckt. Durch diese Abdeckung wird ein problemloses Einführen der Kanüle in die Venen gewährleistet, d.h. beispielsweise ein versehentliches Ankleben der Haltevorrichtung an die Haut des Patienten oder den Handschuhen des Anwenders wird damit verhindert. Bevorzugt ist die Abdeckung, bevorzugt aus Papier, so gefaltet, dass die Abdeckung gefaltet ist und über den Klebestreifen übersteht. Damit wird erreicht, dass sie gut gegriffen und abgezogen werden kann. Anschließend kann die Haltevorrichtung mit einem leichten nach unten gerichteten Druck auf die Haut des Patienten aufgeklebt werden. Somit ist die Haltevorrichtung erst fixiert, nachdem die Sicherheitskanüle in der richtigen Stellung platziert wurde. Nach Beendigung des Dialysevorgangs wird auf die Punktionsstelle ein Tupfer gedrückt und die Kanüle gezogen.

In einer bevorzugten Ausführungsform ist ein Verbindungselement/Kopplung zwischen dem Gehäuse der Haltevorrichtung, in welcher de Sicherheitseinrichtung gelagert ist und einem Schlauch trennbar ausgebildet. In diesem Fall kann der Anwender an dem Schlauch ziehen und die Kanüle aus der Vene zurückziehen. Das Schutzelement wird dabei distal an die Spitze der Kanüle positioniert und umgreift sie am distalen Ende. Anschließend kann die Haltevorrichtung bevorzugt an ihren Flügeln gegriffen werden und mit einem Zug, bevorzugt senkrecht zur Nadelachse, von der Haut des Patienten abgezogen werden und anschließend sicher entsorgt werden, da die Spitze der Sicherheitskanüle von dem Schutzelement umgeben ist. Das Schutzelement hat in diesem Fall nicht ausgelöst, sondern befindet sich noch im Gehäuse der Haltevorrichtung.

Falls während der Behandlung ein unbeabsichtigtes Entfernen der Kanüle erfolgt, beispielsweise durch eine proximale Bewegung des Schlauches, wird ebenfalls durch die Trennung des Verbindungselements vom Gehäuse der Haltevorrichtung das Schutzelement in den zweiten Zustand, also in die Schutzposition, bewegt. Damit wird erreicht, dass der Anwender vor Nadelstichverletzungen geschützt ist und aufgrund des erhöhten Strömungswiderstandes ein Druckanstieg innerhalb des Gerätes zur Behandlung eines extrakorporalen Blutaustauschs registriert und der Blutfluss gestoppt wird.

Bevorzugt weist die Sicherheitseinrichtung eine erste, bevorzugt proximal zu dem Schutzelement angeordneten, Feder auf, mithilfe derer das Schutzelement aus der ersten in die zweite Position bewegt werden kann. In Bezug auf die Haltevorrichtung und/oder der Sicherheitseinrichtung wird somit das Schutzelement distal von dem ersten Zustand (Bereitstellungsposition) in den zweiten Zustand (Schutzposition) bewegt. Insbesondere bewegt die erste Feder allein das Schutzelement. Bevorzugt ist die vorliegende Sicherheitskanüle frei von einem weiteren, nicht durch die erste Feder betätigbaren Schutzelement. Bevorzugt ist die erste Feder in der Bereitstellungsposition, also im ersten Zustand, am proximalen Ende der Sicherheitseinrichtung angeordnet.

Bevorzugt handelt es sich bei dieser ersten Feder um einen Schraubenfeder. Es ist ferner bevorzugt, dass die Sicherheitseinrichtung eine zweite Feder (Rastfeder) aufweist, die dazu geeignet ist, sich beim Ablösen der Haltevorrichtung von der Haut und/oder dem Bewegen der Haltevorrichtung gegenüber der definierten Position automatisch zu entspannen und dadurch die erste Feder freizugeben (d.h. Aufgabe der ersten Rastposition und Scharfschalten der Sicherheitseinrichtung). Bei der zweiten Feder handelt es sich bevorzugt um eine Blattfeder oder eine kegelförmige Schraubenfeder. Alternativ oder zusätzlich dazu weist die zweite Feder bevorzugt einen oder mehrere Rasthaken auf, der/die mit der ersten Feder unmittelbar oder mittelbar in Eingriff tritt/treten.

Erfindungsgemäß muss der zweite, geschlossene Zustand nicht notwendigerweise vollkommen geschlossen sein. Vielmehr kann das Schutzelement im zweiten Zustand mindestens einen oder mehrere Spalte und/oder Öffnungen aufweisen. Dann ist es jedoch bevorzugt, dass der Strömungswiderstand von menschlichem Blut durch sämtliche Spalte und/oder Öffnungen größer ist als der Strömungswiderstand durch das Kanülenlumen. Dies bewirkt, dass im Falle des Auslösens der Sicherheitseinrichtung der Blutfluss gehemmt wird, was zu einem detektierbaren Anstieg des Drucks führt, sodass beispielsweise ein Dialysegerät automatisch abschalten kann. Hierfür ist es auch bevorzugt, dass die Summe der Querschnittsflächen aller Spalte und/oder Öffnungen kleiner ist als die Querschnittsfläche des Kanülenlumens. Ferner ist es bevorzugt, dass der Strömungswiderstand von menschlichem Blut durch sämtliche Spalte und/oder Öffnungen um so viel größer ist als der Strömungswiderstand durch das Kanülenlumen, dass zur Aufrechterhaltung eines konstanten Blutflusses zwischen 300 ml/min und 600 ml/min ein Druckanstieg von mindestens 10 mmHg, bevorzugt von mindestens 30 mmHg und besonders bevorzugt von mindestens 50 mmHg erforderlich ist.

Bevorzugt weist die Haltevorrichtung ein Anschlussstück für einen Schlauch auf oder ist integral mit einem Schlauch verbunden. Bevorzugt ist die Kanüle gemeinsam mit dem Schlauch drehbar in der Haltevorrichtung gelagert.

Die erfindungsgemäße Sicherheitskanüle lässt sich einfach und kostengünstig herstellen, gewährleistet ein hohes Maß an Sicherheit gegenüber Verletzungen und löst die eingangs erwähnten Probleme im Zusammenhang mit Dialysekanülen.

Schließlich betrifft die vorliegende Erfindung nach einem weiteren, ggf. unabhängig beanspruchbaren Aspekt die Bereitstellung eines Blutstopps bzw. Blutstopp-Einrichtung, der vorzugsweise mit einer Sicherheitskanüle, insbesondere gemäß der vorliegenden Erfindung eine (mechanisch starre) Einheit bilden kann, aufweisend einen Klemm-/Quetschkörper, der gegen einen flexiblen Schlauch (mittels einer Feder) vorgespannt ist und einem mit dem Klemm-/Quetschkörper in Wirkeingriff stehenden oder bringbaren Auslösehebel oder Auslöseknopf (der am/im Gehäuse des Blutstopps gelagert ist), der so angeordnet ist, dass er bei bzw. durch Aufbringen des Blutstopps (dessen Gehäuses) auf einer Oberfläche, vorzugsweise einer Patientenhaut in eine erste Position bewegt wird (durch Aufdrücken des Gehäuses auf die Oberfläche), in welcher er den Klemm-/Quetschkörper vom Schlauch beabstandet und der sich bei Abheben des Klemm-/Quetschkörpers von der Oberfläche selbsttätig (aufgrund einer Federvorspannung) in eine zweite Position bewegt, in welcher der Klemm-/Quetschkörper mit dem Schlauch in Quetscheingriff kommt (und diesen sperrt).

Bevorzugte Ausführungsformen der erfindungsgemäßen Sicherheitskanüle werden nachfolgend mit Bezug auf die Figuren im Detail beschrieben. Es zeigen:
Fig. 1 eine Draufsicht auf eine Sicherheitskanüle gemäß einer bevorzugten Ausführungsform mit dem Schutzelement in der ersten Position;
Fig. 2 eine Draufsicht auf die Sicherheitskanüle der Figur 1 mit dem Schutzelement in der zweiten Position;
Fig. 3 eine Seitenansicht der Sicherheitskanüle gemäß Figur 1;
Fig. 4 eine Seitenansicht der Sicherheitskanüle gemäß Figur 2;
Fig. 5 eine seitliche Schnittansicht der Sicherheitskanüle gemäß Figur 1;
Fig. 5A, 5B seitliche Schnittansichten der Sicherheitskanüle in alternativen Ausgestaltungen in der ersten Rastposition;
Fig. 6 eine seitliche Schnittansicht der Sicherheitskanüle gemäß Figur 2;
Fig. 6A, 6B seitliche Schnittansichten der Sicherheitskanüle gemäß der Fig. 5A, 5B jedoch in der zweiten Rastposition;
Fig. 7 eine seitliche Schnittansicht eines Schutzelements gemäß einer bevorzugten Ausführungsform im ersten Zustand;
Fig. 8 eine seitliche Schnittansicht des Schutzelements gemäß Figur 7 im zweiten Zustand;
Fig. 9 eine Draufsicht auf eine ausgestanzte Metallfolie, aus dem ein erfindungsgemäßes Schutzelement gefertigt werden kann;
Fig. 10 eine Teilschnittansicht von oben eines Schutzelements gemäß einer bevorzugten Ausführungsform;
Fig. 11 eine seitliche Schnittansicht des Schutzelements gemäß Figur 10;
Fig. 12 eine seitliche Schnittansicht eines Schutzelements gemäß einer weiteren bevorzugten Ausführungsform;
Fig. 12A eine seitliche Schnittansicht eines Schutzelements gemäß einer weiteren bevorzugten Ausführungsform;
Fig. 13 einen Querschnitt durch die Punktionskanüle und die Haltevorrichtung der erfindungsgemäßen Sicherheitskanüle;
Fig. 14A, 14B jeweils eine seitliche Schnittansicht eines Schutzelements gemäß einer weiteren bevorzugten Ausführungsform in der ersten und der zweiten Rastposition;
Fig. 15A, 15B jeweils eine seitliche Schnittansicht eines Schutzelements gemäß einer weiteren bevorzugten Ausführungsform in der ersten und der zweiten Rastposition;
Fig. 16 eine seitliche Schnittansicht einer (zusätzlichen Schlauchklemme) gemäß einer ersten bevorzugten Ausführungsform der vorliegenden Erfindung;
Fig. 17 eine seitliche Schnittansicht einer (zusätzlichen) Schlauchklemme gemäß einer zweiten bevorzugten Ausführungsform der vorliegenden Erfindung und
Fig. 18A und 18B zeigen ein Entenschnabelventil zur Montage/Anordnung am die Sicherheitseinrichtung aufnehmenden Gehäuse der Haltevorrichtung.

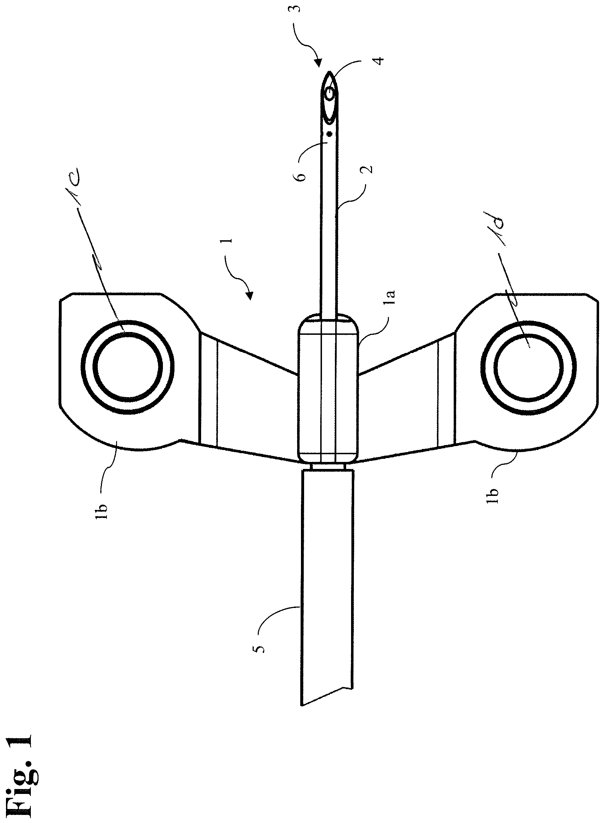

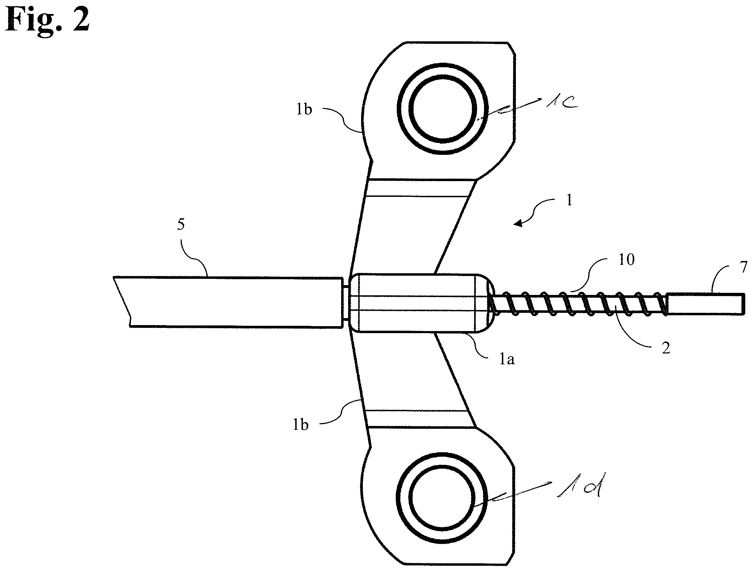

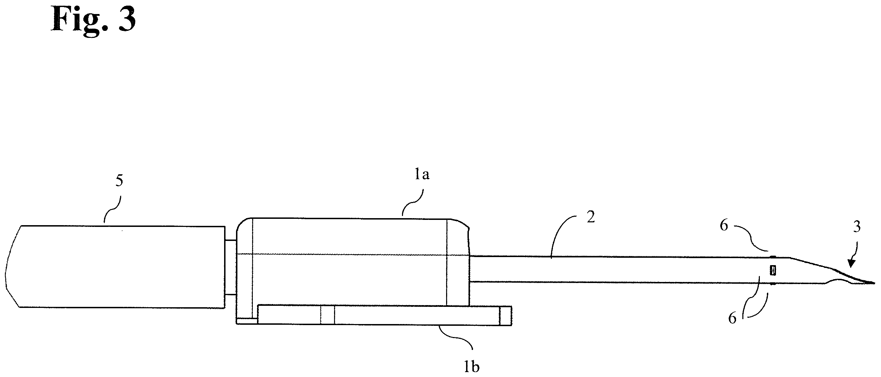

Die Figuren 1 bis 6 zeigen unterschiedliche Ansichten einer Sicherheitskanüle gemäß einer bevorzugten Ausführungsform der vorliegenden Erfindung, wobei das Schutzelement in den Figuren 1, 3 und 5 in der ersten Position ist und in den Figuren 2, 4 und 6 in der zweiten Position ist. Die Sicherheitskanüle weist eine Punktionskanüle 2 mit einem Kanülenlumen 4 und einer angeschliffenen Spitze 3 auf. Die Punktionskanüle 2 ist mit einem Schlauch 5 verbunden. Ferner umfasst die Sicherheitskanüle eine Haltevorrichtung 1, die dazu geeignet, vorzugsweise dazu angepasst ist, während des Gebrauchs in einer definierten Position an der Haut eines Patienten anzuliegen und die Punktionskanüle 2 zu halten. In der dargestellten bevorzugten Ausführungsform umfasst die Haltevorrichtung 1 ein Gehäuse 1a sowie zwei Griffflügel 1b, welche über einen ringförmigen Vorsprung 1c bzw. eine ringförmige Nut 1d miteinander in Eingriff treten können, wenn die beiden Griffflügel 1b hochgeklappt werden. Bevorzugt wird die Sicherheitseinrichtung gegen ein Auslösen blockiert, wenn die beiden Griffflügel 1b nach oben geklappt sind. Hierfür kann beispielsweise an den beiden Griffflügeln 1b oder auch nur an einem der beiden Griffflügel 1b beispielsweise ein Zapfen oder Dorn ausgebildet sein (nicht weiter dargestellt), der mit dem Schutzelement 7 in (Formschluss) Eingriff kommt und dieses in der ersten Position arretiert.

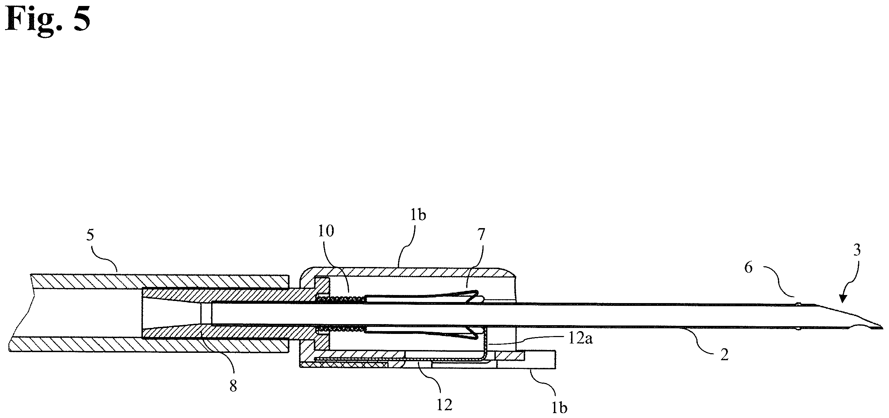

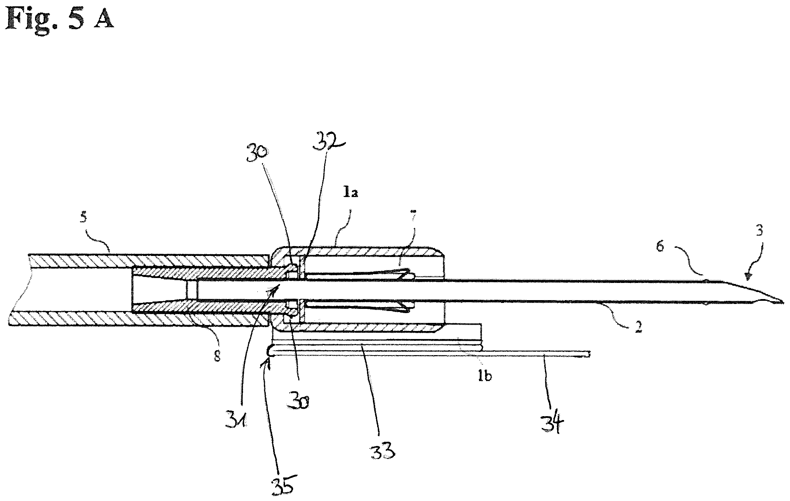

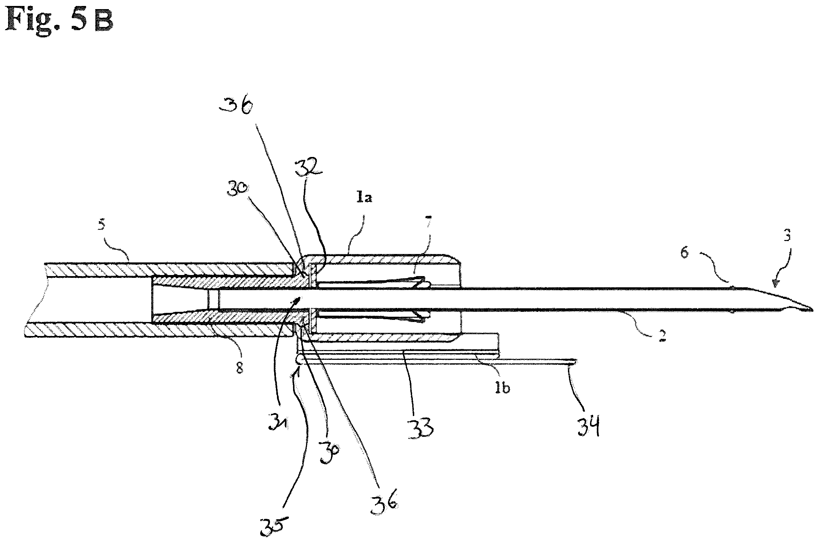

Die Sicherheitseinrichtung weist das vorstehend genannte Schutzelement 7 auf, das vorliegend Hülsen-; Gehäuse- bzw. Klammer-förmig ausgebildet ist und welches einen ersten, geöffneten Zustand (vgl. Figur 5) und einen zweiten, geschlossenen Zustand (vgl. Figuren 2, 4 und 6) einnehmen kann. Wird die Haltevorrichtung 1 von der Haut abgelöst und/oder gegenüber der definierten Position bewegt, so bewegt die Sicherheitseinrichtung 1 automatisch das Schutzelement 7 aus einer ersten Position, in der die Spitze der Kanüle freiliegt (vgl. Figuren 1, 3 und 5), in eine zweite Position, in der die Spitze der Kanüle abgedeckt ist (vgl. Figuren 2, 4 und 6). Dabei geht gleichzeitig das Schutzelement 7 automatisch vom ersten, geöffneten Zustand in den zweiten, geschlossenen Zustand über.

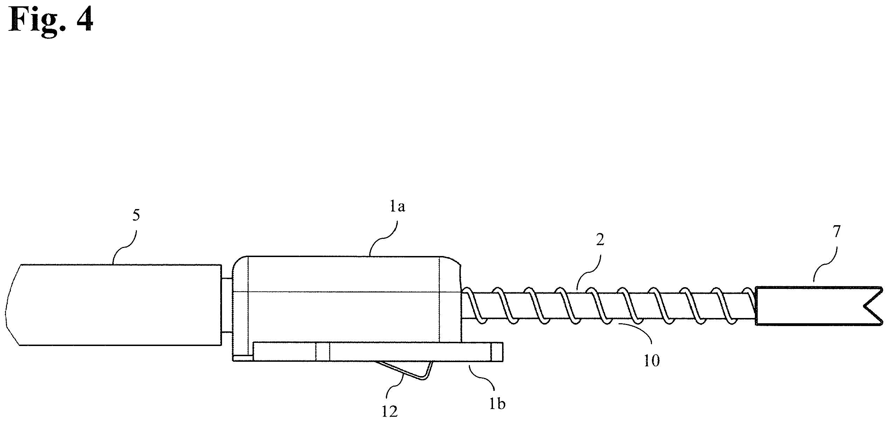

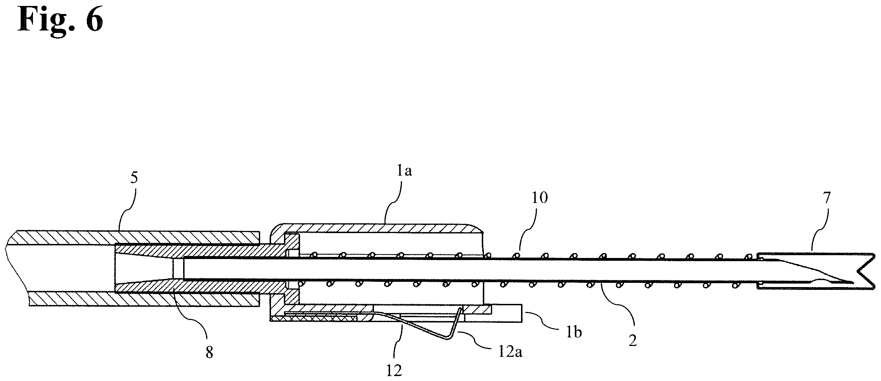

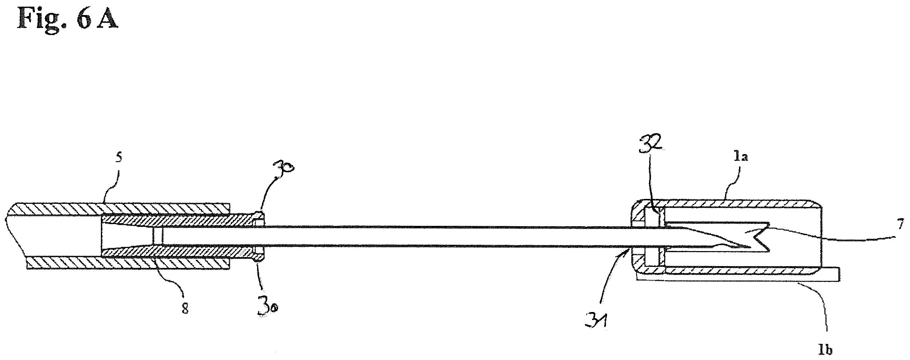

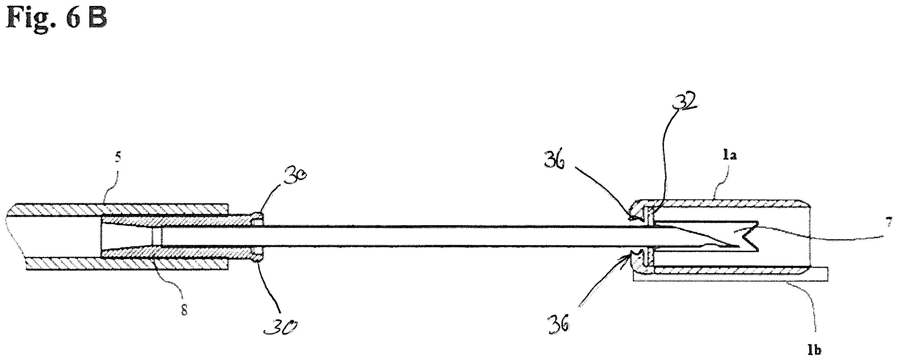

In der in den Figuren 1 bis 6 dargestellten bevorzugten Ausführungsform beruht dieser automatische Sicherheitsmechanismus darauf, dass die Sicherheitseinrichtung 1 eine erste Feder 10 aufweist, mithilfe derer das Schutzelement 7 aus der ersten in die zweite Position bewegt werden kann, und dass die Sicherheitseinrichtung 1 eine zweite Feder 12 aufweist, die dazu geeignet ist, sich beim Ablösen der Haltevorrichtung 1 von der Haut und/oder dem Bewegen der Haltevorrichtung 1 gegenüber der definierten Position automatisch zu entspannen und dadurch die erste Feder 10 freizugeben. Bei der ersten Feder 10 handelt es sich in der dargestellten bevorzugten Ausführungsform um eine Schraubenfeder, deren proximales Ende an einem Verbindungselement 8 anliegt bzw. mit diesem verbunden ist (vgl. Figuren 5 und 6), während deren distales Ende mit dem proximalen Ende des Schutzelements 7 verbunden ist. Befindet sich das Schutzelement in der ersten Position, so ist die Schraubenfeder 10 entgegen ihrer Federspannung komprimiert. Das Schutzelement 7 wird dabei in der ersten Position blockiert, da die zweite Feder 12 einen oder mehrere Rasthaken 12a aufweist, der/die mit der ersten Feder 10 unmittelbar oder mittelbar in Eingriff tritt/treten. In der hier dargestellten bevorzugten Ausführungsform blockiert das Rastelement 12a das distale Ende des Schutzelements 7 und tritt somit mittelbar mit der ersten Feder 10 in Eingriff. Im Falle der hier dargestellten bevorzugten Ausführungsform handelt es sich bei der zweiten Feder 12 um eine Blattfeder, die, während die Haltevorrichtung an der Haut eines Patienten anliegt, entgegen ihrer Federspannung nach innen (d.h. in den Figuren 5 und 6 nach oben) gedrückt wird. Löst sich die Haltevorrichtung 1 von der Haut des Patienten, kann die zweite Feder 12 von dem Gehäuse 1a zurückfedern, wie dies in den Figuren 4 und 6 dargestellt ist, sodass der Rasthaken 12a das Schutzelement 7 (und dadurch mittelbar die erste Feder 10) freigibt. Dies erlaubt der Schraubenfeder 10, sich selbsttätig zu expandieren, sodass deren distales Ende das Schutzelement 7 in distaler Richtung in die zweite Position bewegt, in der die Spitze der Kanüle abgedeckt ist (vgl. Figuren 2, 4 und 6).

Das vorzugsweise hakenförmige Rastelement 12a kann während des Transports in der Verpackung und/oder beim Abnehmen der Schutzkappe der Kanüle 2 und/oder beim Zusammenbringen der Flügel 1b auslösen. Ebenfalls nachteilig wäre, wenn das Rastelement 12a so stark mit dem Schutzelement in Eingriff steht, dass der Sicherheitsmechanismus nicht automatisch oder selbstaktivierend ausgelöst werden kann. Dementsprechend muss die Schutzelementbewegungsvorrichtung, bevorzugt die erste Feder, und/oder das Rastelement 12a selbst so ausgebildet sein, dass ein versehentliches Entrasten oder ein ungewolltes Verrastenbleiben nicht erfolgt. Je nach Kanülenlänge ist daher eine andere erste und/oder zweite Feder, bevorzugt eine unterschiedliche Länge und/oder Federkraft, erforderlich, die so aufeinander abgestimmt sind, dass das federelastischen Rastelement 12a selbsttätig auslöst, jedoch beispielsweise mittels eines einfachen Klebstreifens gesichert werden kann.

In der hier dargestellten bevorzugten Ausführungsform weist das Schutzelement 7 zwei federnde Arme auf, die das Schutzelement 7 im zweiten Zustand verschließen. Im ersten Zustand werden diese beiden Arme entgegen ihrer Federkraft dadurch auf Abstand gehalten, dass sich zwischen den beiden Armen die Kanüle 2 befindet (vgl. Figur 5). Wird das Schutzelement 7 aus der ersten Position durch die Spiralfeder 10 in die zweite Position bewegt, so gleiten die Arme des Schutzelements 7 über die Außenseite der Kanüle 2, bis das distale Ende der Kanüle 7 distal von der Kanülenspitze angeordnet ist. Sobald die Kanüle nicht weiter verhindert, dass die beiden Arme nach innen federn, federn diese aufeinander zu und verschließen so das Schutzelement 7.

Um zu verhindern, dass sich das Schutzelement 7 von der Kanüle 2 löst bzw. dass die Schraubenfeder 10 das Schutzelement 7 in distaler Richtung bis über die Kanülenspitze hinausbewegt, sind bevorzugt ein oder mehrere Vorsprünge 6 am distalen Ende der Kanüle 2 vorgesehen, mit denen das proximale Ende des Schutzelements 7 in Eingriff tritt. Bevorzugt sind die zwei oder mehreren Vorsprünge 6 in axialer Richtung gesehen an der gleichen Position der Kanüle 2 angeordnet. Alternativ sind die Vorsprünge 6 in axialer Richtung gesehen an verschiedenen Positionen der Kanüle 2 angeordnet. Bevorzugt wird der eine oder die mehreren Vorsprünge 6 durch eine, bevorzugt mechanische, Quetschung erzeugt.

Die Kanüle 2 ist über ein Verbindungselement 8 mit dem Schlauch 5 verbunden, wobei das Verbindungselement 8 bevorzugt drehbar in/an dem Gehäuse 1a gelagert ist und vorzugsweise nicht lösbar mit dem Gehäuse 1a gekoppelt ist.

In den Figuren 5A und 6A wird eine weitere Ausführungsform der vorliegenden erfindungsgemäßen Sicherheitskanüle gezeigt. Gleiche und funktionsgleiche Elemente sind mit gleichen Bezugszeichen versehen, sodass insofern auf die vorangegangene Beschreibung verwiesen wird. Gemäß der in den Figuren 5A und 6A gezeigten Ausführungsform weist das Verbindungselement 8 am distalen Ende Rastelemente (z.B. Rastnasen/Leiste, etc.) 30 auf, welche in radialer Richtung eine größere räumliche Ausdehnung als die proximale Öffnung 31 des Gehäuses 1a aufweisen. Die Rastelemente haben Schlitze zwischen sich. Das Verhältnis zwischen der Größe der Rastelemente und der Schlitze kann so eingestellt sein, um eine nötige Kraft zum Entrasten auf einen bestimmten Wert zu regeln. Die Rastelemente 30 können auch als Rastring ausgebildet sein. Die Öffnung 31 und die Rastelemente 30 des Verbindungselements 8 sind so ausgebildet, dass bei einer proximalen Bewegung des Schlauches 5 oder des Verbindungselements 8 und gleichzeitiger unveränderten Position des Gehäuses 1a bezüglich der Patientenhaut sich das Gehäuse 1a und das Verbindungselement 8 wie in Figur 6A gezeigt voneinander trennen lassen. Zur Trennung des Verbindungselements 8 und des Gehäuses 1a darf nicht so viel Kraft aufgewendet werden, wie zur Ablösung der Haltevorrichtung 1, insbesondere über eine Klebeschicht 33, von der Haut eines Patienten erforderlich wäre.

Zudem ist proximal zu dem Schutzelement 7 gemäß der Fig. 5A eine Haltewand 32 oder ein Haltering 32 mit einer Öffnung vorgesehen, sodass sich die Kanüle 2 in proximaler Richtung verschieben lässt, jedoch das Schutzelement 7 durch das proximale Verschieben der Kanüle 2 in Richtung der Spitze 3 der Kanüle 2, also distal, verschoben wird und in der zweiten Position die Spitze 3 umgreift. Die Haltewand 32 oder der Haltering 32 (an welcher sich dann die Feder 10 abstützt) ist bevorzugt einstückig mit dem Gehäuse 1A ausgebildet. Alternativ ist die Haltewand 32 oder der Haltering 32 bevorzugt als separates Bauteil ausgebildet. Die Haltewand 32 oder der Haltering 32 wird dann über einen Haltemechanismus (Verspannen, Rasten, etc.) mit dem Gehäuse 1A verbunden. Außerdem weist die Sicherheitskanüle gemäß Figur 5A (im Kontaktbereich der Haltevorrichtung mit der Patientenhaut) eine Klebeschicht 33 auf, mittels derer die Haltevorrichtung 1 auf der Haut des Patienten platziert/angeklebt werden kann. Diese Klebeschicht 33 ist vor Gebrauch und Positionierung der Sicherheitskanüle an der richtigen Stelle zunächst mit einer Abdeckung 34, auch als Abdeckschicht bezeichnet, geschützt, die vollständig die Klebeschicht 33 bedeckt und am proximalen Ende der Klebeschicht eine Faltung 35 aufweist, sodass die Abdeckung 34 in distaler Richtung mit geringem Abstand der Haltevorrichtung zu der Haut des Patienten abgezogen werden kann. Ebenfalls kann die Abdeckvorrichtung 34 derart auf der Klebeschicht 33 aufgebracht werden, dass sie die Faltung 35 am distalen Ende der Klebeschicht 33 aufweist und somit in proximaler Richtung mit geringem Abstand der Haltevorrichtung 1 zu der Haut des Patienten abgezogen werden kann. Eine entsprechende Klebeschicht 33 und Abdeckvorrichtung 34 ist bevorzugt bei jeder erfindungsgemäßen Sicherheitskanüle vor Anbringen der Sicherheitskanüle an dem Patienten vorhanden.

In den Figuren 5B und 6B wird eine weitere Ausführungsform der vorliegenden erfindungsgemäßen Sicherheitskanüle gezeigt. Gleiche und funktionsgleiche Elemente sind mit gleichen Bezugszeichen versehen, sodass insofern auf die vorangegangene Beschreibung verwiesen wird. In dieser Ausführungsform sind die Rastelemente 30, die bevorzugt in Form eines Rastrings ausgebildet sind, mit zur Aufnahme der Rastelemente 30 ausgebildeten, sich in der proximalen Wand des Gehäuses 1a vorhandenen Aufnahmeelementen 36, die bevorzugt in Form einer ringförmigen Nut ausgebildet sind, verrastet. Die Haltewand 32 oder der Haltering 32 ist im Vergleich zu der Ausführungsform gemäß der Figuren 5A und 6A in distaler Richtung verschoben und liegt bündig an der proximalen Wand des Gehäuses 1a an.

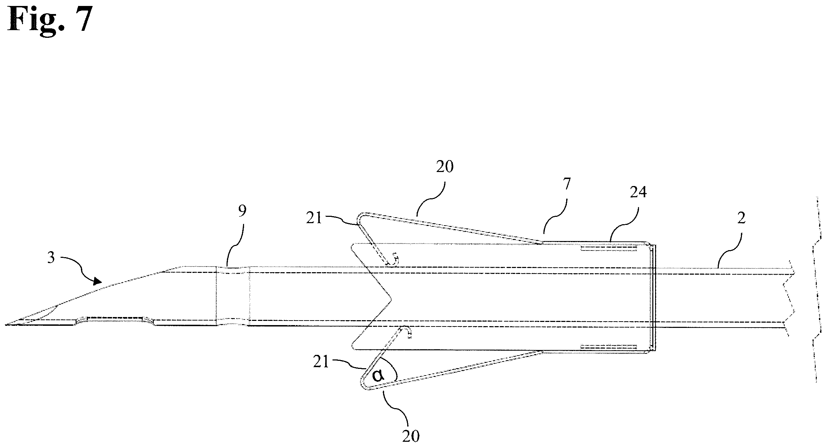

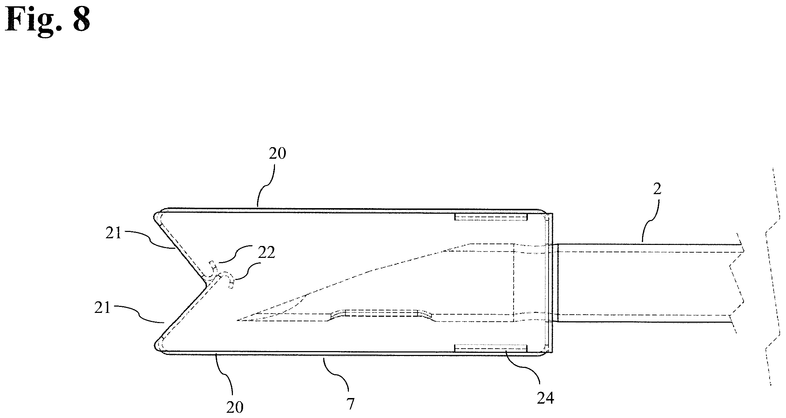

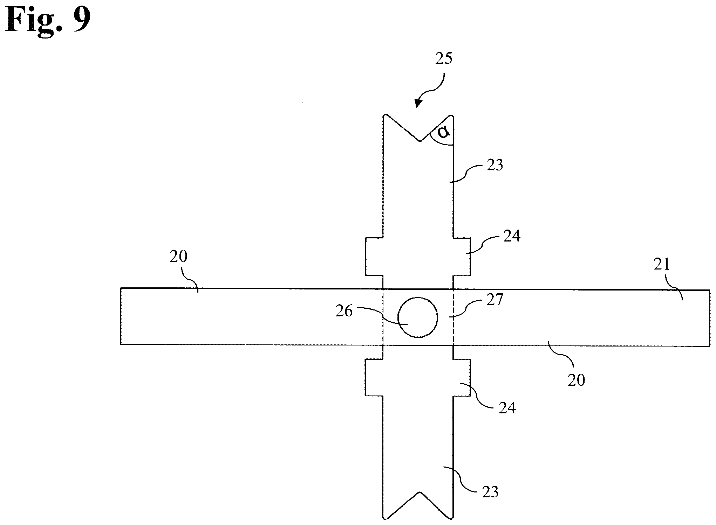

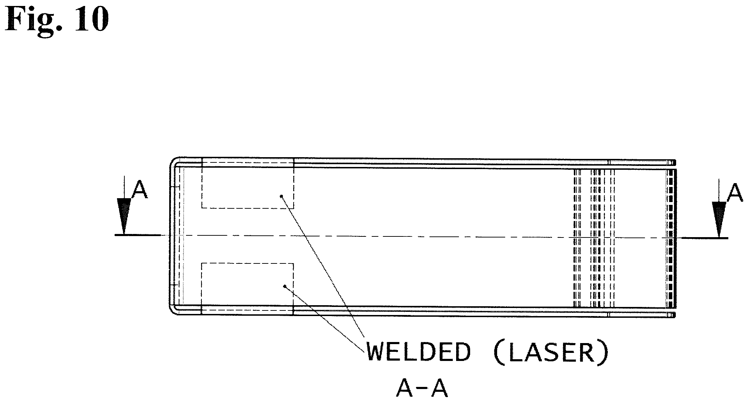

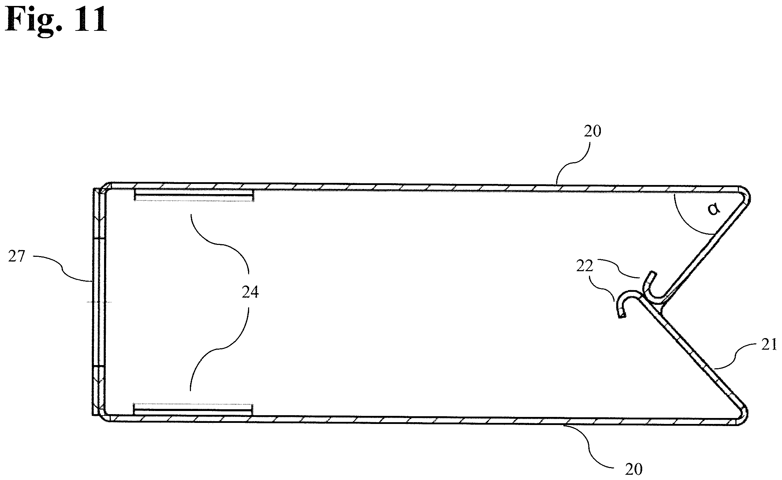

In den Figuren 7-11 ist eine bevorzugte Ausführungsform des erfindungsgemäßen Schutzelements 7 zu sehen. In der hier dargestellten bevorzugten Ausführungsform bildet das Schutzelement 7 ein im Wesentlichen quaderförmiges Gehäuse, welches die Kanülenspitze 3 im geschlossenen Zustand vollständig umschließt bzw. einhüllt. Das Schutzelement 7 weist einen proximalen Wandabschnitt 27 mit einer Öffnung 26 zur Durchführung der Kanüle 2, vier seitliche Wandabschnitte 20 und 23 sowie zwei distale Wandabschnitte 21 auf. Die seitlichen Wandabschnitte 20 sind mit den seitlichen Wandabschnitten 23 mittels Laschen 24 miteinander verschweißt. Die Wandabschnitte 20 können ebenso einteilig mit dem Rest des Bleches ausgestanzt werden. Die beiden seitlichen Wandabschnitte 20 bilden zwei federnde Arme, an deren distalen Ende jeweils ein distaler Wandabschnitt 21 vorgesehen ist. Bei der hier dargestellten bevorzugten Ausführungsform, die aus dem in Figur 9 abgebildeten ausgestanzten Metallblech gebildet ist, können die beiden distalen Wandabschnitte 21 auf in der Blechbearbeitungsindustrie bekannten und üblichen Weise durch Umbiegen bzw. Abknicken der Enden der Abschnitte 20 gewonnen werden. Dabei bilden die Abschnitte 20 und 21 bevorzugt einen spitzen Winkel a, sodass sich die distalen Wandabschnitte 21 ausgehend vom distalen Ende der seitlichen Wandabschnitte 20 sowohl nach innen als auch in proximaler Richtung erstrecken. Dies fördert unter anderem ein reibungsarmes Gleiten des Schutzelements 7 auf der Außenseite der Kanüle 2. Des Weiteren kann ein solcher spitzer Winkel α wirkungsvoll das Zurückziehen des Schutzelements 7 aus der in Figur 8 dargestellten zweiten Position verhindern, da sich bei einem solchen Zurückziehen der durch die Abschnitte 20 und 21 gebildete abgewinkelte Bereich mit der Kanülenspitze 3 verhaken würde. Um diesem spitzen Winkel α Rechnung zu tragen und ein dichtes Verschließen des Schutzelements zu gewährleisten, weisen die distalen Enden der seitlichen Wandabschnitte 23 entsprechende dreieckige Ausnehmungen auf, wobei der in Figur 9 dargestellte Winkel α dem in Figur 11 dargestellten Winkel α entspricht. Der Winkel α kann auch größer sein als der in Figur 11 dargestellte Winkel a, um ein Überlappung hervorzurufen.

Da im Regelfall das Schutzelement 7 aus einem Material (beispielsweise einem Blech bestimmter Dicke) gebildet wird, werden in der Regel auch die seitlichen Wandabschnitte 23 elastisch sein, ohne dass dies jedoch erforderlich wäre, da die Wandabschnitte 23 während des Gebrauchs nicht mit der Kanüle 2 in Kontakt geraten. Um die seitlichen Wandabschnitte 20 elastischer auszubilden als die seitlichen Wandabschnitte 23, können Erstere eine geringere Breite aufweisen als Letztere, wie dies beispielsweise in Figur 9 zu sehen ist. Die Elastizität kann auch über die Wandstärke des Bleches geregelt sein. Bevorzugt ist eine zweiteilige Konstruktion, wie in Figur 9 dargestellt, für den Fall, dass eine unterschiedliche Elastizität gewünscht ist. Bevorzugt ist es, dass die Seitenabschnitte 23 weniger elastisch als die Wandabschnitte 20 sind. Die Elastizität kann insbesondere durch eine Wölbung beeinflusst werden. Insbesondere kann die Elastizität durch eine geprägte Wölbung hervorgerufen werden. Dies ist vorteilhaft, wenn die Seitenabschnitte 23 weniger elastisch sein sollen, bevorzugt in Zusammenhang mit der einteiligen Konstruktion. Auch ist es möglich, Stege/Rippen vorzusehen, welche die Seitenabschnitte 23 aussteifen.

Für den Fall, dass der Winkel α gemäß Fig. 9 größer ist als der Winkel zwischen den Wandabschnitten 20 und 21, kann eine Wölbung der Seitenabschnitte auch das Abdichten zwischen den Seitenabschnitten 20, 21 und 23 verbessern. Die Seitenabschnitte 20 und 21 werden gegen die gewölbten Seitenabschnitte 23 beim Schließen gepresst.

Der zweite, geschlossene Zustand des Schutzelements 7 wird im Wesentlichen dadurch bereitgestellt, dass die seitlichen Wandabschnitte 20 aufgrund der ihnen inhärenten Federkraft bündig an die seitlichen Wandabschnitte 23 angepresst werden. Treffen hier Metallkanten auf Metallkanten, so werden in der Regel kleine Spalte und/oder Öffnungen verbleiben, die jedoch unproblematisch sind, solange der Strömungswiderstand von menschlichem Blut durch sämtliche Spalte und/oder Öffnungen größer ist als der Strömungswiderstand durch das Kanülenlumen. Das Schutzelement 7 kann jedoch besser verschlossen bzw. abgedichtet werden, wenn das Schutzelement 7 zumindest abschnittsweise aus einem elastischen Kunststoff besteht oder mit einem elastischen Kunststoff beschichtet ist. Insbesondere ist es bevorzugt, im Bereich der Kanten bzw. Ränder der seitlichen Wandabschnitte 20 und 23 und/oder der distalen Wandabschnitte 21 einen elastischen Kunststoff vorzusehen. Herstellungstechnisch kann es jedoch einfacher sein, die gesamte Innenseite des Schutzelements (d.h. die in Figur 9 dargestellte Oberfläche) vollständig mit einem elastischen Kunststoff zu beschichten.

Die distalen Wandabschnitte 21 weisen an ihren Innenkanten bevorzugt abgerundete Bereiche und/oder Widerhaken 22 auf. Diese abgerundeten Bereiche 22 verringern einerseits den Gleitwiderstand gegenüber der Außenseite der Kanüle 2 (vgl. Figur 7). Andererseits können diese abgerundeten Bereiche auch als Widerhaken 22 dienen, die das Schutzelement 7 zusätzlich gegen ein proximales Zurückziehen sichern.

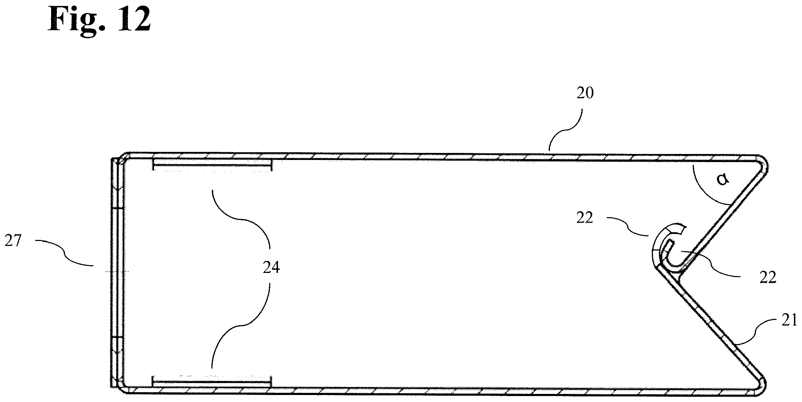

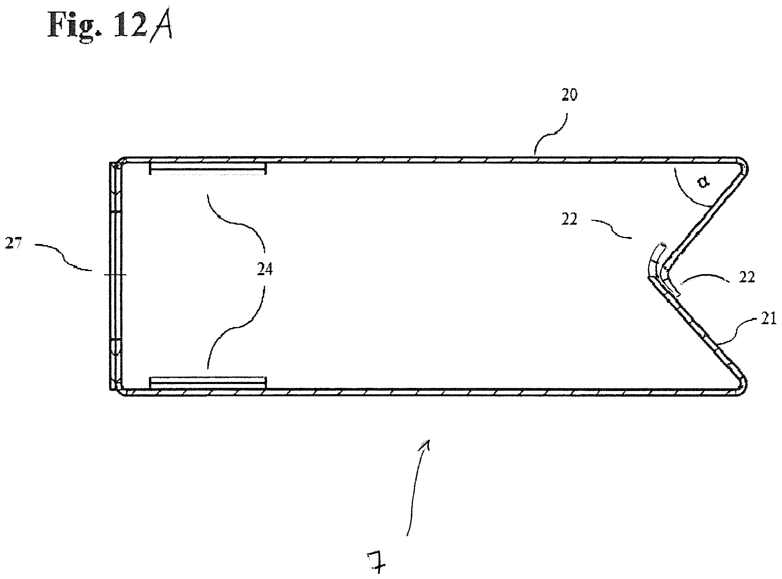

Gemäß einer weiteren bevorzugten Ausführungsform, die schematisch in Figur 12 dargestellt ist, können die Widerhaken 22 derart ausgebildet sein, dass sie sich ineinander verhaken können, so dass die beiden Arme 20 miteinander in Eingriff treten können, wenn das Schutzelement in der zweiten, geschlossenen Position ist. Hierzu können beispielsweise die beiden Widerhaken bzw. abgerundeten Bereiche an den Innenkanten der distalen Wandabschnitte 21 einander umgreifen bzw. umfassen, wie dies in Figur 12 zu sehen ist. Dadurch kann verhindert werden, dass die beiden Arme durch den Druck des Blutes auseinandergedrückt werden können. Allerdings müssen die Funktionen der Sicherung gegen ein Zurückziehen einerseits und gegen ein Auseinanderdrücken andererseits nicht mit Hilfe derselben Elemente 22 bereitgestellt werden. Vielmehr können die beiden Arme zusätzlich zu oder an Stelle von den Widerhaken 22 auch andere Hakenelemente oder gekrümmte Bereiche aufweisen, die in der zweiten Position miteinander in Eingriff treten bzw. sich ineinander verhaken.

Figur 12A zeigt ein Schutzelement 7 mit distalen Wandabschnitten 21, welche an ihrem Ende gebogene Abschnitte 22 aufweisen, wobei die Krümmung der beiden Abschnitte 22 in die gleiche Richtung gekrümmt sind. Durch die Variation des Winkels α und der Krümmung der Widerhaken 22 ist es möglich, einerseits ein Verhaken der Wandabschnitte 21 beim Zusammenbau der Kanüle mit der Schutzeinrichtung zu verhindern und andererseits einen größtmöglichen Widerstand gegen das aus der Kanüle während der Verwendung strömende Blut bereitzustellen, sodass möglichst wenig Blut aus der Sicherheitsvorrichtung strömt.

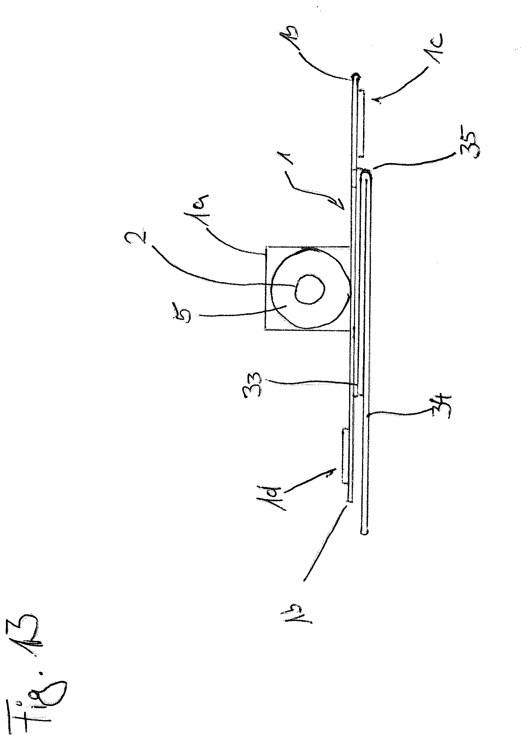

Figur 13 zeigt einen Querschnitt durch die Figur 1. Gleiche und funktionsgleiche Elemente sind mit gleichen Bezugszeichen versehen, sodass insofern auf die vorangegangene Beschreibung verwiesen wird. Die beiden Flügel 1b weisen einen ringförmigen Vorsprung 1c bzw. eine ringförmige Nut 1d auf, welche miteinander in Eingriff treten können. Auf der Unterseite der Haltevorrichtung ist eine Klebeschicht 33 zu sehen, welche vor der Positionierung der Kanüle auf der Haut des Patienten mittels einer Abdeckschicht 34 abgedeckt ist. Im Vergleich zu den Figuren 5A und 5B wird in der Ausführungsform gemäß Figur 13 die Abdeckschicht 34 nicht in axialer Richtung, sondern in radialer Richtung, abgezogen. Die Abdeckung 34 deckt bevorzugt die Klebeschicht 33 vollständig ab. Die Klebeschicht 33 kann bevorzugt auch die komplette Haltevorrichtung 1 abdecken. Die Abdeckung 34 ist bevorzugt einstückig ausgebildet. Bevorzugt kann die Abdeckung auch zweistückig oder mehrstückig ausgebildet sein, bevorzugt derart, dass für jeden Flügel 1B mindestens eine Abdeckung 34 vorhanden ist, die radial nach außen gerichtet abgezogen werden kann.

Selbstverständlich ist die vorliegende Erfindung nicht auf ein Schutzelement gemäß der in den Figuren 7 bis 11 bevorzugten Ausführungsform beschränkt. Das Schutzelement muss nicht aus einem gestanzten Metallblech hergestellt werden, sondern kann beispielsweise auch aus Kunststoff bestehen. Anstelle von zwei distalen Wandabschnitten 21 könnte auch ein einzelner, entsprechend größerer distaler Wandabschnitt 21 vorgesehen sein, der lediglich am distalen Ende eines seitlichen Wandabschnitts 20 vorgesehen ist. Das Schutzelement muss auch keine im Wesentlichen quaderförmige Form aufweisen, sondern kann auch ein Prisma mit beispielsweise dreieckiger, fünfeckiger oder sechseckiger Grundfläche sein. Alternativ kann das Schutzelement auch die Form eines Kreiszylinders haben und beispielsweise aus zwei Halbschalen bestehen. Die seitlichen Wandabschnitte 20 und 23 müssen auch nicht miteinander verschweißt sein, sondern können auch beispielsweise miteinander verklebt sein.

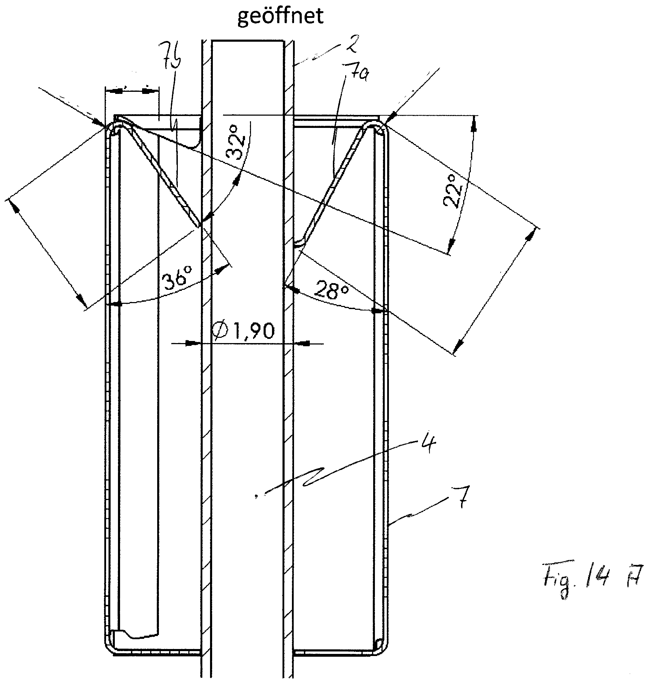

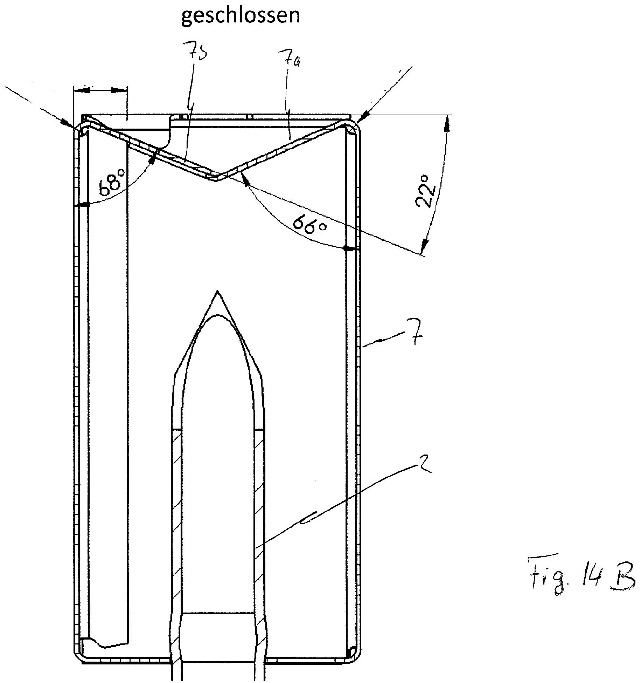

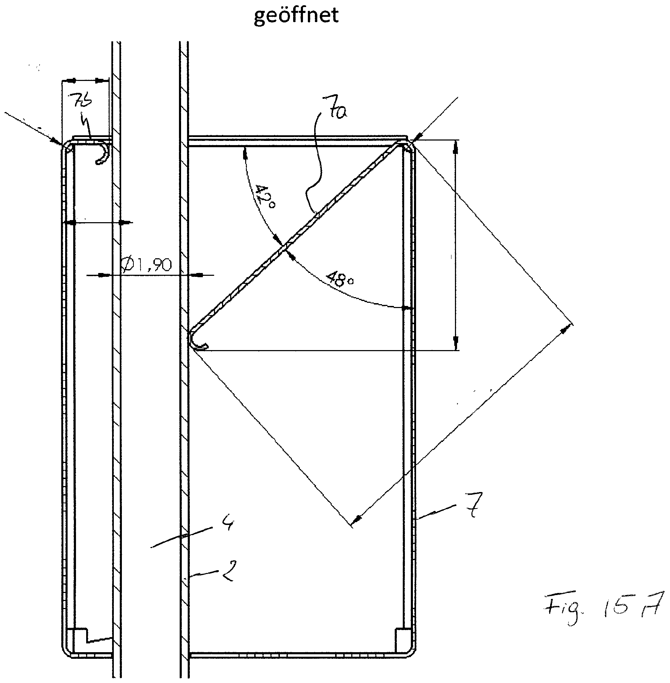

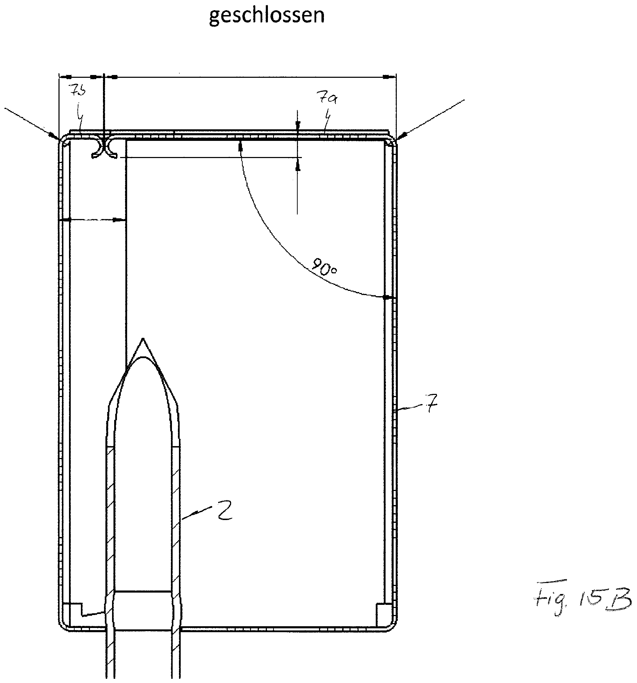

Grundsätzlich sind aber auch andere Ausgestaltungen des Schutzelements denkbar, wofür beispielsweise auf die Ausführungsformen gemäß der Fig. 14A, 14B und 15A, 15B verwiesen wird, wobei die Kanüle auch exzentrisch in dem Schutzelement sitzen kann (15A, 15B).

Demzufolge kann gemäß der Fig. 14A das vorzugsweise Gehäuse-/Klammer-förmige Schutzelement 7 an dessen distalem Endabschnitt mit zwei aufeinander zu schwenkbaren Armen 7a, 7b zueinander unterschiedlicher Armlänge ausgestaltet sein. D.h. einer der Arme 7b ist kürzer als der andere Arm 7a, derart, dass beide Arme 7a, 7b durch die eingeschobene Kanüle 2 um unterschiedliche Winkel (z.B. 22° und 32° bezüglich der Senkrechten zur Kanülenachse) aufgeschwenkt werden. Sobald das Schutzelement 7 mit dem kürzeren Arm 7b über die Kanülenspitze hinaus verschoben wird, schwenkt dieser Arm (zuerst) in seine Schließposition zurück, wobei der längere Arm 7a immer noch vom der Kanüle 2 aufgespreizt wird (bleibt). Sobald dann auch der längere Arm 7a die Kanülenspitze erreicht hat und diese überschreitet, schwenkt dieser ebenfalls in seine Schließposition zurück und versperrt so den Weg für die Kanüle 2 im Wesentlichen dichtend in Richtung Umgebung. Dieser Zustand ist in der Fig. 14B dargestellt. Insoweit ist es möglich, die beiden Arme in Schließposition gemäß der Fig. 14B in vorbestimmter Reihenfolge übereinander zu falten und so ein Durchstoßen der Nadelspitze sicher zu verhindern.

Um ein Überschwenken der zurückfedernden Arme zu vermeiden, kann bevorzugt ein nicht weiter dargestellter Schwenk-/Endanschlag vorgesehen sein, gegen den die zurückfedernden (in Schließposition verschwenkenden) Arme anliegen/anschlagen können.

In der Fig. 15A ist eine Alternative zu der Ausgestaltung gemäß der Fig. 14A dargestellt. Demzufolge ist der eine (kürzere) Arm 7b nicht verschwenkbar (starr), wohingegen der andere (längere) Arm 7a quasi als einflügelige Tür elastisch schwenkbar ausgebildet ist, derart, dass dieser durch die Kanüle 2 radial nach außen aufgeschwenkt werden kann. Sobald das Schutzelement 7 über die Kanülenspitze hinaus axial verschoben wird, schwenkt der eine Arm 7a in seine dichtende Schließposition gemäß der Fig. 15B zurück und sperrt so die Kanülenspitze innerhalb des Schutzelements 7 vorzugsweise dichtend oder weitgehend dichtend ein.

Gemäß der vorstehenden Beschreibung soll durch die Ausbildung der Arme 7a, 7b am Schutzelement 7 auch ein Strömungswiderstand für das aus der Kanüle ausströmende Blut geschaffen werden, der einen Staudruck bewirkt von einer Höhe, die durch eine geeignete Sensorik sicher erfassbar ist und schließlich zur Abschaltung (und/oder Auslösung eines Alarms) der extrakorporalen Blutbehandlungsmaschine führt.

Obgleich dies prinzipiell möglich ist, kann infolge der noch immer vorhandenen BlutLeckage eine gewisse Zeit vergehen, bis sich ein ausreichender Staudruck ausgebildet hat. In der Zwischenzeit ist ein Blutverlust in Kauf zu nehmen.

Um dies zu vermeiden, kann ein sogenannter Blutstopp (Sperrventil) vorgesehen sein, der zusätzlich oder alternativ zu der Dichtfunktion des Schutzelements angeordnet werden kann und daher auch in Kombination mit der erfindungsgemäßen Sicherheitskanüle oder unabhängig hiervon beansprucht werden soll. Insofern ist die nachfolgende Beschreibung des erfindungsgemäßen Blutstopps in Kombination wie auch separat zu der vorstehend beschriebenen Sicherheitskanüle zu sehen. Grundsätzlich hat ein Blutstopp gemäß der vorliegenden Erfindung ein Gehäuse 100, durch welches ein flexibler Blutschlauch 102 hindurchgeführt ist. In dem Gehäuse 100 ist ferner ein Klemm-/Quetschkörper 104 gelagert, der gegen den Blutschlauch 102 federvorgespannt ist. Der Klemm-/Quetschkörper 104 hat eine Eingriffskante/-seite 108, die dafür angepasst ist, den Blutschlauch 102 nahezu fluiddicht abzuquetschen, wofür die auf den Klemm-/Quetschkörper 104 einwirkende Vorspannkraft einer Vorspannfeder 106 ausreicht.

An dem Gehäuse 100 ist ein Auslöseknopf oder Auslösehebel 110 gelagert, der aus dem Gehäuse 100 herausragt bzw. an einer Gehäuseaußenseite gelagert ist und gegen die Gehäuseaußenseite bzw. in das Gehäuseinnere durch eine äußere Kraftbeaufschlagung bewegt werden kann. Der Auslöseknopf oder Auslösehebel 110 ist dabei mit Klemm-/Quetschkörper 104 in Wirkeingriff, derart, dass er diesen in einer ersten, nicht klemmenden/nicht quetschenden Position zurückhält, wenn er in/an das Gehäuse 100 bewegt ist und den Klemm-/Quetschkörper 104 für dessen Bewegen in eine zweite, klemmende/quetschende Position freigibt, wenn er sich aus/weg von dem Gehäuse 100 bewegt.

Dies bedeutet, dass wenn das Gehäuse 100 des Blutstopps beispielsweise an einer Patientenhaut aufliegt - und zwar auf jener Gehäuseseite, an der sich der Auslöseknopf/-hebel 110 befindet - so wird der Auslöseknopf/-hebel 110 gegen/in das Gehäuse 100 gedrückt, wobei der Klemm-/Quetschkörper 104 in der ersten Position gehalten wird, in der Blut frei durch den flexiblen Schlauch 102 innerhalb des Gehäuses 100 strömen kann. Wird hingegen das Gehäuse 100 von der Patientenhaut abgehoben, springt der Auslöseknopf/-hebel 110 vorzugsweise federvorgespannt aus/weg von dem Gehäuse 100 und gibt dabei den Klemm-/Quetschkörper 104 derart frei, dass dieser durch dessen Federvorspannung gegen den im Gehäuse 100 befindlichen flexiblen Schlauch 102 gedrückt wird und diesen sperrt.

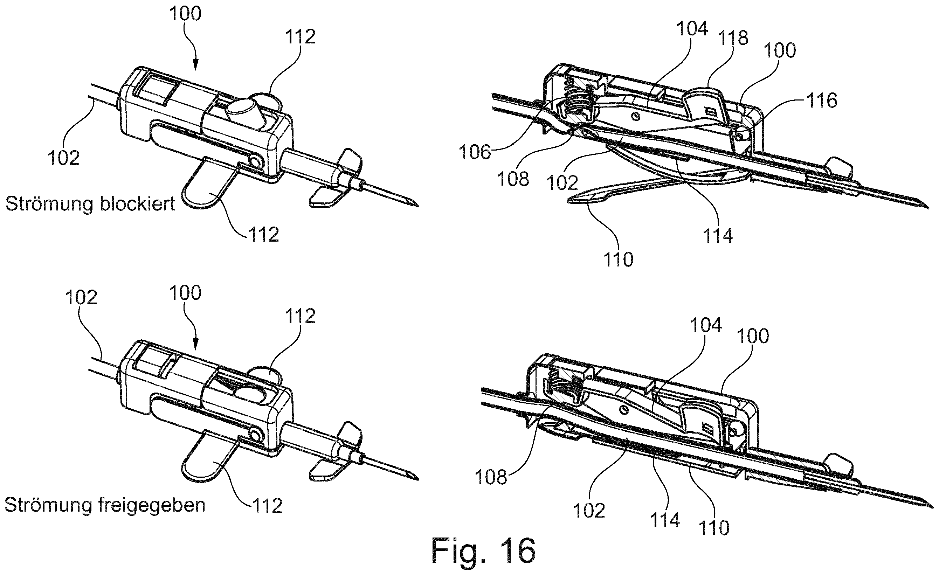

In der Fig. 16 ist eine erste konstruktive Ausführungsform eines Blutstopps gemäß der vorliegenden Erfindung dargestellt.

Demnach hat der erfindungsgemäße Blutstopp das Gehäuse 100, an dem beidseits Patientenhaut-Auflagepads 112 ausgebildet/angeformt sind, deren Patientenhautzugewandte Auflageflächen mit Selbstklebestreifen (nicht dargestellt) versehen sind. Das Gehäuse 100 bildet dabei einen Durchgangskanal 114, in welchen der flexible (Blut-)Schlauch 102 eingelegt bzw. einlegbar ist.

An einer Patientenhaut-zugewandten Gehäuseseite ist der Auslösehebel 110 am Gehäuse 100 angelenkt, an dem ein Verraststift 116 ausgeformt/angeordnet ist, der in das Gehäuse 100 hineinragt und mit dem, vorliegend wippenartig im Gehäuse gelagerten Klemmkörper 104 in Rasteingriff bringbar ist.

Im Konkreten ist ein (dem Verraststift 116 abgewandtes) freies Ende des wippenartigen Klemmkörpers 104 mit der Quetschkante 108 ausgebildet, welche mittels der Feder (Schraubenfeder) 106 gegen den Durchgangskanal 114 bzw. den darin eingelegten Schlauch 102 vorgespannt ist. Das andere freie Ende des wippenartigen Klemmkörpers 104 ist mit einer Rasteinrichtung (z.B. in Form eines Rastvorsprungs, jedoch nicht im Detail dargestellt) ausgeformt, die mit dem Verraststift 116 (z.B. in Form einer Rastnase oder Hakens) in Rasteingriff bringbar ist, um den wippenartigen Klemmkörper 104 in der ersten Position zu halten, in der sich die Quetschkante 108 entfernt vom Durchgangskanal 114 (vom flexiblen Schlauch 102) befindet. Der Rasteingriff ist nur dann möglich, wenn der Auslösehebel 110 gegen die entsprechende Gehäuseseite geschwenkt ist (und damit der Verraststift 116 gegen die Rasteinrichtung gedrückt wird).

Schließlich ist an dem anderen freien Ende des wippenartigen Klemmkörpers 104 (im Bereich der Rasteinrichtung) ein weiterer Betätigungsknopf oder Taste 118 angeordnet/ausgebildet, der aus dem Gehäuse 100 ragt, derart, dass bei dessen manueller Betätigung der wippenartige Klemmkörper 104 aus der zweiten Position, in welcher die Quetschkante 108 gegen den eingelegten Schlauch 102 mittels der Feder 106 gedrückt wird, in die erste Position gegen die Federvorspannung zurückbewegt werden kann, in welcher er mit dem Auslösehebel 110 bzw. dem daran ausgebildeten Raststift 116 in Rasteingriff bringbar ist.

Die Funktion des erfindungsgemäßen Blutstopps lässt sich wie Folgt umschreiben. Zunächst wird das Gehäuse 100 auf eine Patientenhaut aufgeklebt, wodurch der Auslösehebel 110 gegen die Gehäuseaußenseite (federelastisch - siehe die am Ausklösehebel 110 angeordnete Blattfeder gemäß Fig. 16) verschwenkt wird. Anschließend wird der wippenartige Klemmkörper 104 über den Betätigungsknopf 118 in dessen erste Position überführt, in welcher er mit Verraststift 116 am Auslösehebel 110 verrastet. Schließlich wird der flexible Schlauch 102 in den Durchgangskanal 114 eingeschoben.

Sollte nunmehr das Gehäuse 100 des Blutstopps von der Patientenhaut abgelöst/abgehoben werden, schwenkt der Auslösehebel 110 federelastisch von der Gehäuseseite weg, wobei auch der Verraststift 116 mitschwenkt und dabei die Verrastung mit dem wippenartigen Klemmkörper 104 löst. Dieser wird nunmehr infolge der darauf einwirkenden Federkraft durch die Feder 106 ebenfalls wippenartig verschwenkt, wobei dessen Quetschkante 108 gegen den Schlauch 102 gedrückt wird und diesen sperrt.

Sofern der Blutstopp mit der erfindungsgemäßen Sicherheitskanüle vorzugsweise gemäß der vorstehenden Beschreibung gehäuseseitig gekoppelt ist (zu einer, vorzugsweise starren Einheit zusammengebaut oder ausgeformt), würde ein Ablösen der Sicherheitskanüle von der Patientenhaut gleichsam ein Ablösen des Gehäuses 100 des Blutstopps von der Patientenhaut bedeuten, wodurch der vorstehend beschriebene Mechanismus (unabhängig von der Sicherheitskanüle) ausgelöst werden würde.

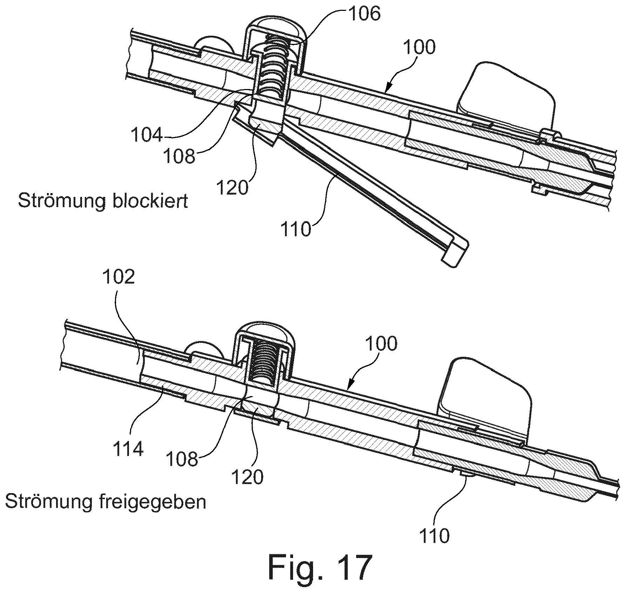

Eine konstruktiv andere Variante des erfindungsgemäßen Blutstopps (Sperrventils) ist in der Fig. 17 dargestellt.

Auch in diesem Fall ist ein Auslösehebel 110 an einer Patientenhaut-zugewandten Seite des Gehäuses 100 des Blutstopps angelenkt. In diesem Fall ist der Klemmkörper 104 aber in Form eines federvorgespannten Stifts ausgebildet, an dessen einer Stirnseite die Vorspannfeder 106 eine axial gerichtete Vorspannkraft aufbringt und an dessen anderer Stirnseite die Quetschkante 108 ausgeformt ist.

In einem (der Feder 106 axial abgewandten) Abschnitt des Stifts 104 ist ein Eingriffselement 120 in der Form einer Halteleiste ausgebildet, an welcher der Auslösehebel 110 nach Art eines Kniehebelmechanismus angreift, um den Stift 104 in die erste Position (weg vom Durchgangskanal 114/eingelegten flexiblen Schlauch 102) zu verschieben, wenn der Auslösehebel 110 gegen die Gehäuseseite verschwenkt wird. Insoweit ist die Funktion des Blutstopps in der Ausführungsform gemäß der Fig. 17 dieselbe, wie die des Ausführungsbeispiels gemäß der Fig. 16 mit der Ausnahme, dass die erste Position des nunmehr stiftförmigen Klemmkörpers 104 beim Ausführungsbeispiel gemäß der Fig. 17 nicht verrastet wird sondern über die Schwenkposition des Auslösehebels 110 sowie die darauf kontinuierlich aufgebrachte Kraft gehalten werden muss.

Die Ausführungsform gemäß der Fig. 17 ist daher nicht notwendiger Weise als konstruktiv einfachere Variante der Ausführungsform gemäß Fig 16 gedacht. Vielmehr wird hier der Effekt angestrebt, dass der Mechanismus nicht erst "scharfschalten" oder "aktivieren" muss, wie dies bei der Ausführungsform gemäß der Fig. 16 der Fall ist.

In anderen Worten ausgedrückt, wenn der Mechanismus / das Gehäuse des Blutstopps auf der Haut eine Patienten klebt, dann wird der Auslösehebel 110 zwangsweise an das Gehäuse 100 des Blutstopps geführt und somit der Teil / das Eingriffselement 120 in das Gehäuse 100 verschoben, um den Blutweg freizugeben. Wird nun das Gehäuse 100 von der Patientenhaut abgelöst, wird durch die Feder 106 das Teil / das Eingriffselement 120 wieder (aus dem Gehäuse 100 heraus) verschoben, sodass kein Blutfluss stattfinden kann. Eine alternative, erfindungsgemäße Idee kann hier sein, dass nicht das Abklemmen eines Schlauches erreicht werden soll, sondern die "Verschiebung" eines Gehäusesegmentes bewirkt wird, das Teil des Blutwegs darstellt und somit wie der Ventilkolben eines Sperrventils der Schiebeventilbauart wirkt. Natürlich muss hierbei die Abdichtung zwischen dem Eingriffselement 120 und dem Gehäuse 100 passen, damit das System funktioniert und es keine Undichtigkeiten gibt.

Eine weitere alternative oder zusätzliche Möglichkeit der Ausbildung/Anordnung eines Blutstopps ist die Ausbildung des Gehäuses der Haltevorrichtung mit einem sogenannten Entenschnabelventil, das auch in Kombination mit der erfindungsgemäßen Sicherheitskanüle oder unabhängig hiervon beansprucht werden soll.

Ein solches Ventil ist beispielsweise in der Fig. 17A und 17B schematisch dargestellt. In diesem Fall ist auch das Gehäuse 1a, wie es bereits vorstehend anhand der Fig. 1 bis 13 beschrieben wurde, mit einer Art Aufstülpkragen 200 (Entenschnabelventil) ausgebildet/versehen, der an der distalen Stirnkante des Gehäuses 1a angeordnet ist und eine ähnliche Funktion hat, wie die schwenkbaren Arme am Schutzelement 7.

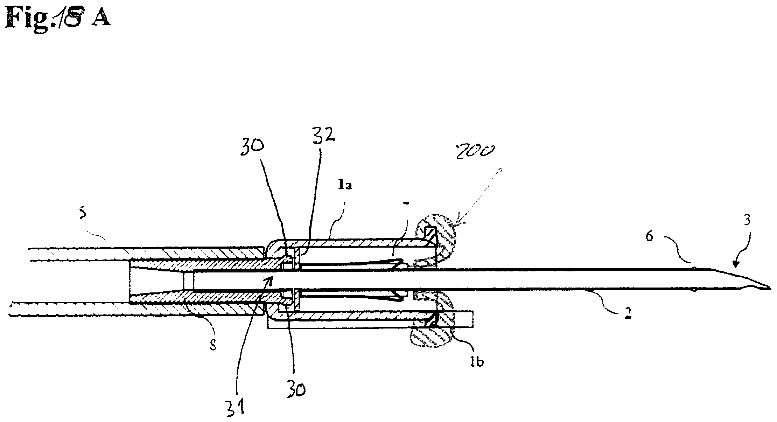

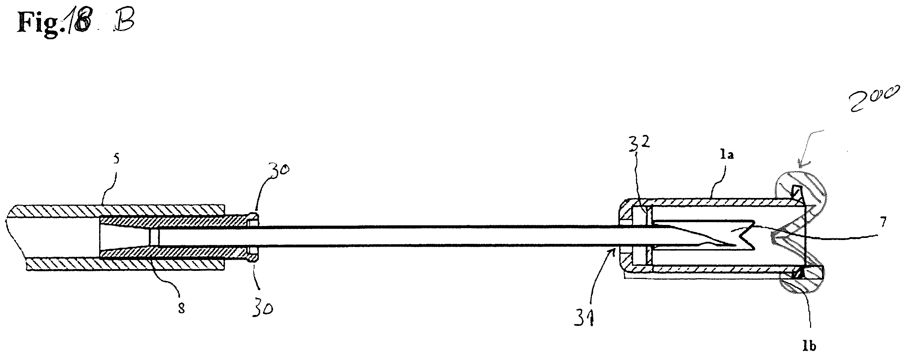

Der Aufstülpkragen 200 wird vorliegend auf den distalen Endabschnitt des Gehäuses der Haltevorrichtung aufgesetzt und verschließt federelastsich das Gehäuse in Richtung distal. Die Punktierkanüle kann nunmehr durch den Aufstülpkragen 200 hindurch unter dessen radialer Aufspreizung hindurchgeschoben werden. Dieser Zustand ist in der Fig. 18A dargestellt.

Sollte nunmehr die Punktierkanüle 2 in das Gehäuse der Haltevorrichtung zurückgezogen werden (Schutzelement 7 hat bereits ausgelöst und verschließt die Spitze der Punktierkanüle 2) so verschließt der Aufstülpkragen das Gehäuse und verhindert so weitgehend eine Blutleckage.

## Patentansprüche

1. Sicherheitskanüle, insbesondere Dialysekanüle, mit:
einer Punktionskanüle (2) mit einem Kanülenlumen (4) und einer Spitze (3),
einer Haltevorrichtung (1), die dazu geeignet ist, während des Gebrauchs in einer definierten Position an der Haut eines Patienten anzuliegen und die Punktionskanüle (2) zu halten und
einer Sicherheitseinrichtung mit einem Schutzelement (7), welches einen ersten, geöffneten Zustand und einen zweiten, geschlossenen Zustand einnehmen kann;
wobei die Sicherheitseinrichtung dazu geeignet ist, das Schutzelement (7) aus einer ersten Position, in der die Spitze (3) der Kanüle (2) freiliegt, in eine zweite Position, in der die Spitze der Kanüle abgedeckt ist, zu bewegen, wodurch gleichzeitig das Schutzelement (7) vom ersten, geöffneten Zustand in den zweiten, geschlossenen Zustand übergeht,
**dadurch gekennzeichnet, dass**
das Schutzelement (7) im zweiten Zustand Spalte und/oder Öffnungen aufweist und der Strömungswiderstand von menschlichem Blut durch sämtliche Spalte und/oder Öffnungen größer ist als der Strömungswiderstand durch das Kanülenlumen (4).

2. Sicherheitskanüle nach Anspruch 1, wobei die Sicherheitseinrichtung eine erste Feder (10) aufweist, mit Hilfe derer das Schutzelement (7) aus der ersten in die zweite Position bewegt werden kann.

3. Sicherheitskanüle nach Anspruch 2, wobei die Sicherheitseinrichtung eine zweite Feder (12) aufweist, die dazu geeignet ist, sich bei Ablösen der Haltevorrichtung (1) von der Haut und/oder Bewegen der Haltevorrichtung (1) gegenüber einer definierten Position automatisch zu entspannen und dadurch die erste Feder (10) freizugeben.

4. Sicherheitskanüle nach Anspruch 3, wobei die zweite Feder (12) eine Blattfeder oder eine kegelförmige Schraubenfeder ist und/oder wobei die zweite Feder (12) einen oder mehrere Rasthaken (12a) aufweist, der/die mit der ersten Feder (10) unmittelbar oder mittelbar in Eingriff tritt/treten.

5. Sicherheitskanüle nach einem der vorhergehenden Ansprüche, wobei das Schutzelement (7) im zweiten Zustand Spalte und/oder Öffnungen aufweist und wobei der Strömungswiderstand von menschlichem Blut durch sämtliche Spalte und/oder Öffnungen um so viel größer ist als der Strömungswiderstand durch das Kanülenlumen (4), dass zur Aufrechterhaltung eines konstanten Blutflusses zwischen 300 ml/min und 600 ml/min ein Druckanstieg von mindestens 10 mmHg, bevorzugt von mindestens 30 mmHg und besonders bevorzugt von mindestens 50 mmHg erforderlich ist.

6. Sicherheitskanüle nach einem der vorhergehenden Ansprüche, wobei das Schutzelement (7) im zweiten Zustand Spalte und/oder Öffnungen aufweist und wobei die Summe der Querschnittsflächen aller Spalte und/oder Öffnungen kleiner ist als die Querschnittsfläche des Kanülenlumens (4).

7. Sicherheitskanüle nach einem der vorhergehenden Ansprüche, wobei das Schutzelement (7) zwei federnde Arme (20) aufweist, die das Schutzelement (7) im zweiten Zustand verschließen.

8. Sicherheitskanüle nach Anspruch 7, wobei die Arme (20) derart ausgebildet sind, dass sie im ersten Zustand reibungsarm über die Außenseite der Kanüle (2) gleiten können.

9. Sicherheitskanüle nach Anspruch 7 oder 8, wobei mindestens einer der Arme (20) an seinem distalen Ende eine Arretiereinrichtung aufweist, die verhindert, dass das Schutzelement (7) aus der zweiten Position in die erste Position bewegt wird.

10. Sicherheitskanüle nach Anspruch 9, wobei die Arretiereinrichtung einen abgewinkelten Abschnitt (21) des Arms (20) und/oder einen Widerhaken (22) aufweist.

11. Sicherheitskanüle nach einem der Ansprüche 7 bis 10, wobei die zwei Arme (20) dazu geeignet sind, miteinander in Eingriff zu treten, bevorzugt miteinander zu verrasten und/oder sich ineinander zu verhaken, wenn das Schutzelement (7) in der zweiten, geschlossenen Position ist.

12. Sicherheitskanüle nach-einem der vorhergehenden Ansprüche, wobei das distale Ende des Schutzelements (7) konkav ausgebildet ist.

13. Sicherheitskanüle nach einem der vorhergehenden Ansprüche, wobei das Schutzelement (7) zumindest abschnittsweise aus einem elastischen Kunststoff besteht oder mit einem elastischen Kunststoff beschichtet ist, wobei der elastische Kunststoff bevorzugt eines oder eine Kombination der folgenden Materialien aufweist: Silikon, Polyurethan, PTFE.

14. Sicherheitskanüle nach einem der vorhergehenden Ansprüche, wobei die Innenseite des Schutzelements (7) zumindest abschnittsweise mit einem gerinnungsfördernden Material beschichtet ist, wobei das gerinnungsfördernde Material bevorzugt eines oder eine Kombination der folgenden Materialien aufweist: Proteine wie Kollagen, Fibrin, Thrombin; Polypeptide wie Gelatine; Polysacharide wie Cellulose, Zucker; Glucosamine wie Chitosan; Alginate; Adsorbierende Substanzen wie Zeolith, Aluminophosphat; Denaturierende Substanzen wie Aldehyde, Alaun, Aluminiumsalze.

15. Sicherheitskanüle nach einem der vorhergehenden Ansprüche, wobei das Schutzelement (7) auf der Innenseite eines oder eine Kombination der folgenden Materialien aufweist: elastischer Schaumstoff wie Schaumstoff aus Polyurethan oder Silikon; Quellstoffe; schwammartige und/oder wasserabsorbierende Substanzen.

16. Sicherheitskanüle nach einem der vorhergehenden Ansprüche, wobei die Kanüle (2) einen oder mehrere Vorsprünge (6) aufweist, der/die verhindern, dass sich das Schutzelement (7) von der Kanüle (2) löst.

17. Sicherheitskanüle nach einem der vorhergehenden Ansprüche, ferner mit einem Verbindungselement, bevorzugt mit einem Faden, welches das Schutzelement (7) mit dem Kanülenansatz der Kanüle (2) verbindet.

18. Sicherheitskanüle nach einem der vorhergehenden Ansprüche, wobei an der Haltevorrichtung (1) eine lösbare Einrichtung, bevorzugt ein Klebestreifen und/oder ein lösbarer Clip und/oder Haltegriff, vorgesehen ist, um ein Auslösen des Sicherheitsmechanismus vor Gebrauch der Sicherheitskanüle zu verhindern.

19. Sicherheitskanüle nach einem der vorstehenden Ansprüche, **dadurch gekennzeichnet, dass** die Haltevorrichtung (1) ein Gehäuse zur darin Aufnahme der Sicherheitseinrichtung hat oder ausbildet, das in Richtung distal derart offen ist, um ein Bewegen des Schutzelements (7) aus dem Gehäuse heraus in Richtung Kanülenspitze zu ermöglichen, wobei diese Öffnung mit einem Aufstülpkragen (200) elastisch verschlossen ist, der von der Punktierkanüle (2) aufgespreizt wird.

20. Sicherheitskanüle nach einem der vorstehenden Ansprüche, **gekennzeichnet durch** einen Blutstopp mit einem Klemm-/Quetschkörper, der gegen einen flexiblen Schlauch vorgespannt ist und einem mit dem Klemm-/Quetschkörper in Wirkeingriff stehenden oder bringbaren Auslösehebel oder Auslöseknopf, der so angeordnet ist, dass er bei/durch Aufbringen des Blutstopps auf einer Oberfläche, vorzugsweise einer Patientenhaut in eine erste Position bewegt wird, in welcher er den Klemm-/Quetschkörper vom Schlauch beabstandet und der sich bei Abheben des Klemm-/Quetschkörpers von der Oberfläche selbsttätig in eine zweite Position bewegt, in welcher der Klemm-/Quetschkörper mit dem Schlauch in Quetscheingriff kommt.

## Claims

1. A safety needle, in particular a dialysis needle, comprising:
a puncture needle (2) having a needle lumen (4) and a tip (3),
a holding device (1) which is suitable, during use, for bearing in a defined position on the skin of a patient and for holding the puncture needle (2) and a safety mechanism with a protection element (7) which can adopt a first, opened state and a second, closed state;
wherein the safety mechanism is suitable to move the protection element (7) from a first position, in which the tip (3) of the needle (2) is exposed, to a second position, in which the tip of the needle is covered, as a result of which the protection element (7) at the same time transfers from the first, opened state to the second, closed state,
**characterized in that**
the protection element (7) comprises gaps and/or openings in the second state and wherein the flow resistance of human blood through all gaps and/or openings is larger than the flow resistance through the needle lumen (4).

2. The safety needle according to claim 1, wherein the safety mechanism comprises a first spring (10) by means of which the protection element (7) can be moved from the first position to the second position.

3. The safety needle according to claim 2, wherein the safety mechanism comprises a second spring (12) which, when the holding device (1) is taken away from the skin and/or when the holding device (1) is moved with respect to a defined position, is suitable to relax automatically and thus release the first spring (10).

4. The safety needle according to claim 3, wherein the second spring (12) is a leaf spring or a conical coil spring and/or wherein the second spring (12) comprises one or more latching hooks (12a) entering into engagement with the first spring (10) in direct or indirect manner.

5. The safety needle according to any of the preceding claims, wherein the protection element (7) comprises gaps and/or openings in the second state and wherein the flow resistance of human blood through all gaps and/or openings is larger than the flow resistance through the needle lumen (4) by such an amount that maintaining a constant blood flow of between 300 ml/min and 600 ml/min requires a pressure increase of at least 10 mmHg, preferably of at least 30 mmHg and especially preferred of at least 50 mmHg.

6. The safety needle according to any of the preceding claims, wherein the protection element (7) comprises gaps and/or openings in the second state and wherein the sum of the cross-sectional areas of all gaps and/or openings is smaller than the cross-sectional area of the needle lumen (4).

7. The safety needle according to any of the preceding claims, wherein the protection element (7) comprises two resilient arms (20) which close the protection element (7) in the second state.

8. The safety needle according to claim 7, wherein the arms (20) are formed such that in the first state they are able to slide along the outer side of the needle (2) with low friction.

9. The safety needle according to claim 7 or 8, wherein at least one of the arms (20) has its distal end provided with an arresting device which prevents the protection element (7) from being moved from the second position to the first position.

10. The safety needle according to claim 9, wherein the arresting device comprises a bent portion (21) of the arm (20) and/or a barb (22).

11. The safety needle according to any of claims 7 to 10, wherein the two arms (20) are suitable to come into engagement with each other, preferably to interlock with each other and/or to get entangled with each other if the protection element (7) is in the second, closed position.

12. The safety needle according to any of the preceding claims, wherein the distal end of the protection element (7) has a concave design.

13. The safety needle according to any of the preceding claims, wherein the protection element (7) is made of an elastomer or coated with an elastomer at least in parts, wherein the elastomer comprises preferably one of the following materials or a combination of the following materials: silicone, polyurethane, PTFE.

14. The safety needle according to any of the preceding claims, wherein the inner side of the protection element (7) is coated at least in parts with a coagulatory material, wherein the coagulatory material comprises preferably one of the following materials or a combination of the following materials: proteins such as collagen, fibrin, thrombin; polypeptides such as gelatin; polysaccharides such as cellulose, sugars; glucosamines such as chitosan; alginates; adsorbing substances such as zeolites, aluminophosphate; denaturing substances such as aldehydes, alum, aluminum salts.

15. The safety needle according to any of the preceding claims, wherein the protection element (7) has the inner side provided with one of the following materials or a combination of the following materials: elastic foamed plastic such as a foamed plastic made of polyurethane or silicone; swelling agents; spongy and/or water-absorbing substances.

16. The safety needle according to any of the preceding claims, wherein the needle (2) comprises one or more protrusions (6) preventing the protection element (7) from detaching from the needle (2).

17. The safety needle according to any of the preceding claims, further comprising a connecting element, preferably with a thread, connecting the protection element (7) to the needle attachment of the needle (2).

18. The safety needle according to any of the preceding claims, wherein the holding device (1) is provided with a detachable means, preferably an adhesive strip and/or a detachable clip and/or hand grip, in order to prevent the safety mechanism from being activated before use of the safety needle.

19. The safety needle according to any of the preceding claims, **characterized in that** the holding device (1) comprises or forms a housing for receiving the safety mechanism therein, which is open in distal direction so as to allow a movement of the protection element (7) out of the housing and toward the needle tip, wherein said opening is elastically closed by an everting collar (200) which is spread apart by the puncture needle (2).

20. The safety needle according to any of the preceding claims, **characterized by** a blood stopping means comprising a clamping/pinching body which is biased against a flexible hose and a release lever or release button which is in operative engagement or can be brought into operative engagement with the clamping/pinching body, said release lever or release button being arranged such that during/by applying the blood stopping means on a surface, preferably the patient's skin, it is moved to a first position in which it keeps the clamping/pinching body spaced from the hose and during lifting the clamping/pinching body from the surface automatically moves to a second position in which the clamping/pinching body pinches off the hose.

## Revendications

1. Canule de sécurité, en particulier canule de dialyse, avec :
une canule de ponction (2) avec une lumière de canule (4) et une pointe (3),
un dispositif de maintien (1), qui est adapté pour reposer pendant l'utilisation dans une position définie contre la peau d'un patient et pour maintenir la canule de ponction (2), et
un système de sécurité avec un élément de protection (7), qui peut adopter un premier état ouvert et un deuxième état fermé ;
dans laquelle le système de sécurité est adapté pour déplacer l'élément de protection (7) depuis une première position, dans laquelle la pointe (3) de la canule (2) est dégagée, dans une deuxième position, dans laquelle la pointe de la canule est recouverte,
ce qui a pour effet de manière simultanée que l'élément de protection (7) passe du premier état ouvert dans le deuxième état fermé,
**caractérisée en ce que**
l'élément de protection (7) présente dans le deuxième état des fentes et/ou des ouvertures et la résistance à l'écoulement du sang humain à travers toutes les fentes et/ou ouvertures est plus grande que la résistance à l'écoulement à travers la lumière de canule (4).

2. Canule de sécurité selon la revendication 1, dans laquelle le système de sécurité présente un premier ressort (10), à l'aide duquel l'élément de protection (7) peut être déplacé depuis la première dans la deuxième position.

3. Canule de sécurité selon la revendication 2, dans laquelle le système de sécurité présente un deuxième ressort (12), qui est adapté pour se détendre automatiquement lors du décollement du dispositif de maintien (1) de la peau et/ou déplacement du dispositif de maintien (1) par rapport à une position définie et pour ainsi dégager le premier ressort (10).

4. Canule de sécurité selon la revendication 3, dans laquelle le deuxième ressort (12) est un ressort à lames ou un ressort hélicoïdal de forme conique et/ou dans laquelle le deuxième ressort (12) présente un ou plusieurs crochets d'enclenchement (12a), qui vient/viennent en prise directement ou indirectement avec le premier ressort (10).

5. Canule de sécurité selon l'une quelconque des revendications précédentes, dans laquelle l'élément de protection (7) présente dans le deuxième état des fentes et/ou ouvertures et dans laquelle la résistance à l'écoulement du sang humain à travers toutes les fentes et/ou ouvertures est d'autant plus élevée que la résistance à l'écoulement à travers la lumière de canal (4) que pour conserver un débit de sang constant entre 300 ml/min et 600 ml/min, une augmentation de pression d'au moins 10 mmHg, de manière préférée d'au moins 30 mmHg et de manière particulièrement préférée d'au moins 50 mmHg, est requise.

6. Canule de sécurité selon l'une quelconque des revendications précédentes, dans laquelle l'élément de protection (7) présente dans le deuxième état des fentes et/ou ouvertures et dans laquelle la somme des surfaces de section transversale de toutes les fentes et/ou ouvertures est inférieure à la surface de section transversale de la lumière de canule (4).

7. Canule de sécurité selon l'une quelconque des revendications précédentes, dans laquelle l'élément de protection (7) présente deux bras (20) à ressorts, qui ferment l'élément de protection (7) dans le deuxième état.

8. Canule de sécurité selon la revendication 7, dans laquelle les bras (20) sont réalisés de telle manière qu'ils peuvent glisser dans le premier état avec peu de frottements sur le côté extérieur de la canule (2).

9. Canule de sécurité selon la revendication 7 ou 8, dans laquelle au moins un des bras (20) présente au niveau de son extrémité distale un système d'arrêt, qui empêche que l'élément de protection (7) ne soit déplacé depuis la deuxième position dans la première position.

10. Canule de sécurité selon la revendication 9, dans laquelle le système d'arrêt présente une section (21) coudée du bras (20) et/ou une barbe (22).

11. Canule de sécurité selon l'une quelconque des revendications 7 à 10, dans laquelle les deux bras (20) sont adaptés pour venir en prise l'un avec l'autre, de manière préférée pour s'enclencher l'un avec l'autre et/ou s'accrocher l'un dans l'autre quand l'élément de protection (7) est dans la deuxième position fermée.

12. Canule de sécurité selon l'une quelconque des revendications précédentes, dans laquelle l'extrémité distale de l'élément de protection (7) est réalisée de manière concave.

13. Canule de sécurité selon l'une quelconque des revendications précédentes, dans laquelle l'élément de protection (7) est constitué au moins par endroits d'une matière synthétique élastique ou est revêtu d'une matière synthétique élastique, dans laquelle la matière synthétique élastique présente de manière préférée un matériau ou une combinaison des matériaux suivants : silicone, polyuréthane, PTFE.

14. Canule de sécurité selon l'une quelconque des revendications précédentes, dans laquelle le côté intérieur de l'élément de protection (7) est revêtu au moins par endroits d'un matériau favorisant la coagulation, dans laquelle le matériau favorisant la coagulation présente de préférence un matériau ou une combinaison des matériaux suivants : protéines telles que du collagène, de la fibrine, de la thrombine ; polypeptides tels que des gélatines ; polysaccharides tels que de la cellulose, du sucre ; glucosamines telles que du chitosan ; alginates ; substances adsorbantes telles que de la zéolithe, du phosphate d'aluminium ; substances dénaturantes telles que des aldéhydes, de l'alun, des sels d'aluminium.

15. Canule de sécurité selon l'une quelconque des revendications précédentes, dans laquelle l'élément de protection (7) présente sur le côté intérieur un matériau ou une combinaison de matériaux suivants : mousse élastique telle que de la mousse composée de polyuréthane ou de silicone ; matières de gonflement ; substances spongieuses et/ou absorbant l'eau.

16. Canule de sécurité selon l'une quelconque des revendications précédentes, dans laquelle la canule (2) présente une ou plusieurs parties faisant saillie (6), qui empêche/empêchent que l'élément de protection (7) ne se détache de la canule (2).

17. Canule de sécurité selon l'une quelconque des revendications précédentes, en outre avec un élément de liaison, de manière préférée avec un fil, qui relie l'élément de protection (7) à l'appendice de canule de la canule (2).

18. Canule de sécurité selon l'une quelconque des revendications précédentes, dans laquelle est prévu au niveau du dispositif de maintien (1) un système amovible, de manière préférée une bande adhésive et/ou un clip amovible et/ou une poignée de maintien, pour empêcher un détachement du mécanisme de sécurité avant l'utilisation de la canule de sécurité.

19. Canule de sécurité selon l'une quelconque des revendications précédentes, **caractérisée en ce que** le dispositif de maintien (1) a ou réalise un boîtier servant à y recevoir le système de sécurité, qui est ouvert dans la direction distale de telle manière pour permettre un déplacement de l'élément de protection (7) hors du boîtier en direction de la pointe de canule, dans laquelle ladite ouverture est fermée de manière élastique avec un rebord rabattable (200), qui est écarté par la canule de ponction (2).

20. Canule de sécurité selon l'une quelconque des revendications précédentes,
**caractérisée par** un arrêt du sang avec un corps de serrage/d'écrasement, qui est précontraint contre un tuyau flexible, et un levier de déclenchement ou un bouton de déclenchement coopérant ou pouvant coopérer avec le corps de serrage/d'écrasement, qui est disposé de telle sorte qu'il est déplacé, lors/du fait de l'installation de l'arrêt de sang sur une surface, de préférence la peau d'un patient, dans une première position, dans laquelle il espace le corps de serrage/d'écrasement du tuyau et qui se déplace, lors du soulèvement du corps de serrage/d'écrasement, de la surface de manière autonome dans une deuxième position, dans laquelle le corps de serrage/d'écrasement vient en prise par écrasement avec le tuyau.
